# EUROPÄISCHE PATENTSCHRIFT

(11) **EP 2 779 830 B1**
(45) Veröffentlichungstag und Bekanntmachung des Hinweises auf die Patenterteilung: **17.05.2017**
(21) Anmeldenummer: 12790521.4
(22) Anmeldetag: 15.11.2012
(51) Int. Cl.: A01N 25/22, A01N 47/12, C09D 167/08, C08L 79/02, C09D 5/14, C08G 73/02

(54) **STABILISIERUNG VON IOD ENTHALTENDEN VERBINDUNGEN MIT STICKSTOFF ENTHALTENDEN POLYMEREN**
STABILIZATION OF COMPOUNDS CONTAINING IODINE HAVING POLYMERS COMPRISING NITROGEN
STABILISATION DE COMPOSÉS CONTENANT DE L'IODE AVEC DES POLYMÈRES CONTENANT DE L'AZOTE

(30) Priorität: 16.11.2011 EP 11189405
(43) Veröffentlichungstag der Anmeldung: 24.09.2014
(73) Patentinhaber: LANXESS Deutschland GmbH, 50569 Köln (DE)
(72) Erfinder: BÖTTCHER, Andreas, 50859 Köln (DE); UHR, Hermann, 51373 Leverkusen (DE); SPETMANN, Peter, 51371 Leverkusen (DE); JAETSCH, Thomas, 50668 Köln (DE); FÜHR, Jörg, 47829 Krefeld (DE)
(86) Internationale Anmeldenummer: PCT/EP2012/072760
(87) Internationale Veröffentlichungsnummer: WO 2013/072427

(56) Entgegenhaltungen:
- EP-A1- 2 236 033
- EP-A1- 2 270 087
- WO-A1-97/24390
- WO-A1-2007/101549
- WO-A2-2008/016837

## Beschreibung

Die Erfindung betrifft die Herstellung von Stickstoff enthaltenden Polymeren aus Aziridinen, die Verwendung dieser Stickstoff enthaltenden Polymeren zur Stabilisierung Iod enthaltender Verbindungen, Zusammensetzungen enthaltend zumindest die Stickstoff enthaltenden Polymere sowie Iod enthaltende Verbindungen sowie den Einsatz dieser Zusammensetzungen als Biozide bzw. zur Bekämpfung von Mikroorganismen.

Iod enthaltende Biozide werden zum Schutz technischer Materialien wie zum Beispiel Anstrichmitteln vor dem Befall, der Zersetzung, Zerstörung und optischen Veränderung durch Pilze, Bakterien und Algen eingesetzt. Darüber hinaus werden Iod enthaltende Biozide, auch in Kombination mit Bioziden anderer Wirkstoffklassen, als Komponenten biozid wirksamer Materialschutzmittel wie beispielsweise Holzschutzmittel eingesetzt. Neben Iodalkinylverbindungen werden hier auch Wirkstoffe eingesetzt, in denen ein oder mehrere Iodatome an sp²-hybridisierte Kohlenstoffatome von olefinischen Doppelbindungen, aber auch an sp³-hybridisierte Kohlenstoffatome gebunden sind.

Vielen Iod enthaltenden Bioziden ist gemeinsam, dass sie sich unter Lichteinwirkung selbst in Substanz oder als Komponente eines technischen Materials unter Gelbfärbung zersetzen, was sowohl die biozide Ausstattung als auch die Haptik des zu schützenden Materials massiv beeinträchtigt.

Viele Iod enthaltende Biozide, insbesondere Iodalkinylverbindungen, werden durch Übergangsmetallverbindungen besonders rasch zerstört. Diese Tatsache verhindert den Einsatz von Iod enthaltenden Bioziden, wie insbesondere Iodalkinylverbindungen in lösungsmittelbasierten Anstrichmitteln, wie Farben, Lacken und Lasuren, oder bioziden Schutzmitteln, wie Holzschutzgrundierungen, Holzschutzimprägnierungen und Holzschutzlasuren, da diese Alkydharzbasierten Beschichtungs- und Schutzsysteme typischerweise Übergangsmetallverbindungen enthalten. Die Übergangsmetallverbindungen wie beispielsweise Cobalt-, Blei-, Mangan- und Vanadiumoctoate, fungieren hier als Trockner (Sikkative) des Alkydharz-haltigen Bindemittelsystems. Darüber hinaus werden Übergangsmetallverbindungen auch als farbgebende Pigmente eingesetzt, und weisen mit den Sikkativen vergleichbare destruktive Eigenschaften auf.

Neben den Sikkativen gibt es in den oben genannten Lösungsmittel-basierten Systemen eine Reihe weiterer Bestandteile, die in unterschiedlicher Intensität zu einem Abbau Iod enthaltender Biozide führen. Während bei den üblicherweise verwendeten Lösungsmitteln der destabilisierende Effekt noch relativ schwach ausgebildet ist, zeigen die andere übliche Komponenten einer Farbformulierung, wie beispielsweise Prozessadditive, Weichmacher, Farbpigmente, Antiabsetzmittel, Thixotropiermittel, Korrosionsinhibitoren, Hautverhinderer und Binder einen mehr oder weniger stark ausgeprägten destabilisierenden Effekt.

Neben den oben beschriebenen Lösungsmittel-basierten Systemen ist auch der Einsatz von Iod enthaltenden Bioziden in bestimmten, Wasser-basierten technischen Materialien problematisch. Basiert beispielsweise die Filmbildung und Filmhärtung eines Wasser basierten Anstrichmittels auf der oxidativen Vernetzung wasserlöslicher oder emulgierter Alkyd-Harze, so kommen auch in diesen Systemen Übergangsmetallverbindungen als Sikkative zum Einsatz, womit eine Zerstörung der Iod enthaltenden Biozide einhergeht.

Aus dem Stand der Technik sind Methoden bekannt, den Abbau von Iodpropargylverbindungen in übergangsmetallhaltigen, Lösungsmittel-basierten, Alkyd-Harz enthaltenden Farben zu verhindern und so zu stabilisieren. So ist beispielsweise der Zusatz von Chelatisierungsreagenzien (WO 98/22543 A), organischen Epoxiden (WO 00/16628 A, US 4,276,211, US 4,297,258) gegebenenfalls in Verbindung mit UV-Absorbern (WO 99/29176 A) oder Benzyliden-Campfer-Derivaten (US 6,472,424), Tetraalkylpiperidinverbindungen und/oder UV-Absorbern (EP 0 083 308 A), 2-(2-Hydroxyphenyl)-benzotriazolen (WO 2007/028527 A) oder Azolverbindungen (WO 2007/101549 A) bekannt.

Die Wirkung der oben genannten Stabilisatoren ist jedoch nicht immer ausreichend und mit anwendungstechnischen Nachteilen behaftet. So werden insbesondere Trockenzeiten von Farben deutlich verlängert, was in vielen Fällen für den Anwender nicht akzeptabel ist. Außerdem ist die Inhibierung der Verfärbung nicht immer ausreichend.

In EP 2 236 033 A wird nun die Stabilisierung durch Aziridingruppen enthaltende Stabilisatoren beschrieben. Allerdings lassen sich auf diese Weise keine lagerstabilen Konzentrate von Iod enthaltenden Bioziden erzeugen.

Durch Aufbringen von Aziridinen oder anderer Stickstoff enthaltender Verbindungen auf anorganische Trägermaterialien, wie beispielsweise Kieselsäuren (WO2010/142790 A) lassen sich gute Stabilisatoren erhalten, allerdings ist deren Herstellung aufgrund der erforderlichen Sprühtrocknung sehr energieintensiv.

Es ist weiterhin bekannt, Iod enthaltende Biozide in einer Polymerpräparation vor dem Angriff durch destabilisierende Einflüsse zu schützen (WO 2011/000794 A). Gleichzeitig wird jedoch die Wirksamkeit so eingeschränkt, dass die erforderliche Aufwandmenge unwirtschaftlich hoch wird.

Es bestand daher die Aufgabe, Mittel bereitzustellen, die eine gute Stabilisierung von Iod enthaltenden Verbindungen ermöglichen leicht herstellbar sind und in der Anwendung zum Beispiel in Anstrichmitteln möglichst wenig stören.

Es wurde nun gefunden, dass Stickstoff enthaltende Polymere geeignet sind, Iod enthaltende Verbindungen, insbesondere in (organischen) Lösungsmittel- und Wasser-basierten Systemen sowohl vor chemischem als auch Licht induzierten Abbau wirksam schützen und damit Farbveränderungen und Wirkungsverlust verhindern können.

Die Erfindung betrifft daher die Verwendung von Stickstoff enthaltenden Polymeren, die durch Umsetzung von Aziridinen in Gegenwart von Wasser erhältlich sind, zur Stabilisierung von Iod enthaltenden Verbindungen sowie ein Verfahren zur Stabilisierung von Iod enthaltenden Verbindungen durch Inkontaktbringen mit Stickstoff enthaltenden Polymeren, die durch Umsetzung von Aziridinen in Gegenwart von Wasser erhältlich sind.

Im Rahmen der Erfindung wird unter Stabilisierung wird der Schutz von Iod enthaltenden Verbindungen vor chemischem und/oder Licht induziertem Abbau verstanden.

Iod enthaltende Verbindungen sind beispielsweise Iodalkinylverbindungen sowie Verbindungen, in denen ein oder mehrere Iodatome an sp²-hybridisierte Kohlenstoffatome von olefinischen Doppelbindungen, oder an sp³-hybridisierte Kohlenstoffatome gebunden sind. Vorzugsweise weisen solche Verbindungen biozide Wirkung auf.

Iod enthaltende Verbindungen mit biozider Wirksamkeit sind beispielsweise N-(C₁-C₁₂)-Alkyl-iodtetrazole, N-(C₆-C₁₅)-Aryl-iodtetrazole, N-(C₆-C₁₅)-Arylalkyl-iodtetrazole Diiodmethyl-p-tolylsulfon, Diiodmethyl-p-chlorphenylsulfon, 3-Brom-2,3-diiod-2-propenylalkohol, 2,3,3-Triiodallylalkohol, 4-Chlor-2-(2-chlor-2-methylpropyl)-5-[(6-iod-3-pyridinyl)methoxy]-3(2H)-pyridazinone (CAS-RN: 120955-77-3), Iodfenfos, 3-Iod-2-propinyl-2,4,5-trichlorphenylether, 3-Iod-2-propinyl-4-chlorphenylformal (IPCF), N-Iodpropargyloxycarbonyl-alanin, N-Iodpropargyloxycarbonyl-alanin-ethylester, 3-(3-Iodpropargyl)-benzoxazol-2-on, 3-(3-Iodpropargyl)-6-chlorbenzoxazol-2-on, 3-Iod-2-propinyl-alkohol, 4-Chlorphenyl-3-iodpropargylformal,3-Iod-2-propinyl-propyl-carbamat, 3-Iod-2-propinyl-butyl-carbamat (IPBC), 3-Iod-2-propinyl-m-chlorphenyl-carbamat, 3-Iod-2-propinyl-phenylcarbamat, Di-(3-Iod-2-propinyl)hexyl-dicarbamat, 3-Iod-2-propinyloxyethanol-ethylcarbamat, 3-Iod-2-propinyl-oxyethanol-phenyl-carbamat, 3-Iod-2-propinyl-thioxo-thioethylcarbamat, 3-Iod-2-propinyl-carbaminsäureester (IPC), 3-Brom-2,3-diiod-2-propenylethylcarbamat, 3-Iod-2-propinyl-n-hexylcarbamat und 3-Iod-2-propinyl-cyclohexylcarbamat.

Bevorzugte Iod enthaltende Verbindungen mit biozider Wirksamkeit sind 3-Iod-2-propinyl-2,4,5-trichlorphenylether, 3-Iod-2-propinyl-4-chlorphenylformal (IPCF), N-Iodpropargyloxycarbonyl-alanin, N-Iodpropargyloxycarbonyl-alanin-ethylester, 3-(3-Iodpropargyl)-benzoxazol-2-on, 3-(3-Iodpropargyl)-6-chlorbenzoxazol-2-on, 3-Iod-2-propinyl-alkohol, 4-Chlorphenyl-3-iodpropargylformal, 3-Iod-2-propinyl-propyl-carbamat, 3-Iod-2-propinyl-butyl-carbamat (IPBC), 3-Iod-2-propinyl-m-chlorphenyl-carbamat, 3-Iod-2-propinyl-phenyl-carbamat, Di-(3-Iod-2-propinyl)hexyl-dicarbamat, 3-Iod-2-propinyloxyethanol-ethylcarbamat, 3-Iod-2-propinyl-oxyethanol-phenyl-carbamat, 3-Iod-2-propinyl-thioxo-thioethylcarbamat, 3-Iod-2-propinyl-carbaminsäureester (IPC), 3-Brom-2,3-diiod-2-propenylethylcarbamat, 3-Iod-2-propinyl-n-hexylcarbamat und 3-Iod-2-propinyl-cyclohexylcarbamat.

Besonders bevorzugte Iod enthaltende Verbindungen mit biozider Wirksamkeit sind 3-Iod-2-propinyl-propyl-carbamat, 3-Iod-2-propinyl-butyl-carbamat (IPBC), 3-Iod-2-propinyl-m-chlorphenyl-carbamat, 3-Iod-2-propinyl-phenyl-carbamat, Di-(3-Iod-2-propinyl)hexyl-dicarbamat, 3-Iod-2-propinyloxyethanol-ethylcarbamat, 3-Iod-2-propinyl-oxyethanol-phenyl-carbamat, 3-Iod-2-propinyl-thioxo-thioethylcarbamat, 3-Iod-2-propinyl-carbaminsäureester (IPC), 3-Brom-2,3-diiod-2-propenylethylcarbamat, 3-Iod-2-propinyl-n-hexylcarbamat und 3-Iod-2-propinyl-cyclohexylcarbamat, wobei 3-Iod-2-propinyl-butyl-carbamat (IPBC) noch weiter bevorzugt ist.

Die Stickstoff enthaltenden Polymere besitzen vorzugsweise ein gewichtsmittleres Molekulargewicht von mehr als 1,000 g/mol, vorzugsweise 2,000 bis 100,000 g/mol und besonders bevorzugt 3,000 und 60,000 g/mol bestimmt durch Gel-Permeations-Chromatographie gegen Polystyrol-Standard (sofern nicht anders angegeben: Polystyrol/PSS Polymerkit)

Die Stickstoff enthaltenden Polymere weisen vorzugsweise einen Stickstoffgehalt von 1 bis 20 Gew.-%, bevorzugt 2 bis 15 Gew.-% N, und besonders bevorzugt 5 bis 12 % Gew.-% N ermittelt durch Elementaranalyse auf. Stickstoff enthaltende Polymere sind solche, die Struktureinheiten aufweisen, die sich von Aziridinen ableiten.

Bei der Reaktion von Aziridinen in Gegenwart von Wasser kann der Aziridinring durch nukleophile Reaktion mit Wasser geöffnet werden, wobei ein beta-Aminoalkohol entsteht. Die Aminogruppe selbst kann als starkes Nukleophil dann beispielsweise die nukleophile Ringöffnung eines weiteren Aziridinringes bewirken, wodurch ein Dimer enthaltend eine beta-Aminoamin-Funktion entsteht, die wiederum weiter unter Bildung höherer Polymere reagieren kann.

Vorzugsweise sind Stickstoff enthaltende Polymere deshalb solche, die zumindest eine, vorzugsweise mehrere beta-Aminoamin-Funktionen besitzt,
Bevorzugt sind Aziridinverbindungen der Formel (I) 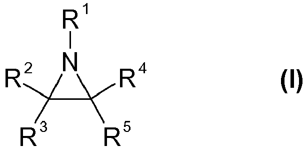 wobei
- R¹: Wasserstoff, Alkyl oder Cycloalkyl, die jeweils unsubstituiert oder substituiert und/oder einfach- oder mehrfach ethylenisch ungesättigt sind, jeweils substituiertes oder unsubstituiertes Fullerenyl, Aryl, Alkoxy, Alkoxycarbonyl, Arylcarbonyl oder Alkanoyl bedeutet,
- R², R³, R⁴ und R⁵: unabhängig voneinander die gleiche Bedeutung wie R¹ haben und zusätzlich unabhängig Halogen, Hydroxyl, Carboxyl, Alkylsulfonyl, Arylsulfonyl, Nitril, Isonitril bedeuten und
- R² und R⁴ oder R³ und R⁵: gemeinsam mit den Kohlenstoffatomen, an die sie gebunden sind, einen 5-bis 10 gliedrigen carbocyclischen Ring bilden, der unsubstituiert oder substituiert und/oder einfach- oder mehrfach ethylenisch ungesättigt ist.

Als monofunktionelle Aziridine der Formel (I) kommen bspw. solche in Frage, worin R² und R⁴ oder R³ und R⁵ gemeinsam mit den Kohlenstoffatomen, an die sie gebunden sind, einen 5- bis 10 gliedrigen carbocyclischen Ring bilden, der unsubstituiert oder substituiert und/oder einfach- oder mehrfach ethylenisch ungesättigt ist.

Insbesondere sind dies solche der Formel (II) 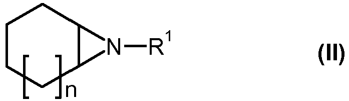 wobei der carbocyclische Ring unsubstituiert ist oder mit einem oder mehreren Substituenten ausgewählt aus der Reihe Halogen, Hydroxyl, Oxo, Carboxyl, Alkylsulfonyl, Arylsulfonyl, Nitril, Isonitril, Alkyl oder Cycloalkyl, die jeweils unsubstituiert oder substituiert und/oder einfach- oder mehrfach ethylenisch ungesättigt sind, substituiertes oder unsubstituiertes Fullerenyl, Aryl, Alkoxy, Alkoxycarbonyl oder Alkanoyl substituiert ist und
n für eine Zahl von 0 bis 6, bevorzugt von 0 bis 1 steht.

Ebenfalls sind solche monofunktionelle Aziridinverbindungen der Formel (I) bevorzugt, worin R¹ für einen Rest der Formel 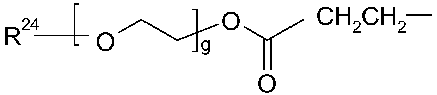 oder 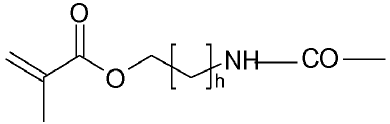 steht,
worin
R²⁴ für -H oder Alkyl, bevorzugt für -H, -CH₃, -C₂H₅, besonders bevorzugt für -CH₃, -C₂H₅ steht,
g eine Zahl von 1 bis 4, bevorzugt 1 bis 3, besonders bevorzugt 1 bis 2 ist,
h eine Zahl von 1 bis 11, bevorzugt 1 bis 5 und besonders bevorzugt 1 bis 3 ist
und die übrigen Reste, die obige Bedeutung haben.

Insbesondere sind solche Verbindungen der Formel (I) bevorzugt, die der Verbindung der Formel (III) oder (IV) entsprechen. 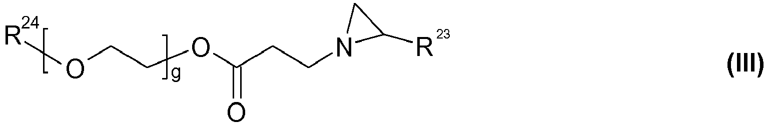 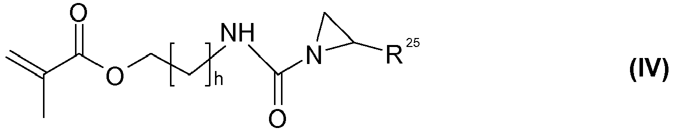 wobei
R²³ für -H oder Alkyl, bevorzugt für -H oder -CH₃, besonders bevorzugt für -CH₃ steht,
R²⁵ für -H oder Alkyl, bevorzugt für -H oder -CH₃, besonders bevorzugt für -CH₃ steht und die übrigen Reste die obige Bedeutung haben.

Besonders bevorzugt sind Aziridine, die zwei oder mehrere Aziridinfunktionen haben. Beispielsweise sind Verbindungen der Formel (V) zu nennen 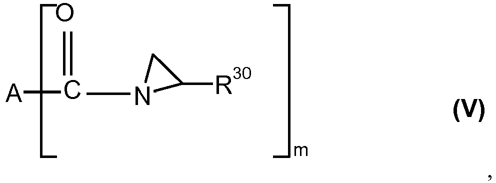 worin
- A: für einen m-valenten aliphatischen, cycloaliphatischen oder aromatischen Rest steht, der ggf. substituiert ist,
- m: für eine Zahl von 2 bis 5, insbesondere 2 bis 3 steht, und
- R³⁰: für jede m-Einheit jeweils unabhängig für Wasserstoff oder C₁-C₄-Alkyl, insbesondere CH₃ oder CH₂CH₃ steht.

Bei m = 2 steht A bevorzugt für C₂-C₁₀-Alkylen,
insbesondere für
-(CH₂)₆)-, -C(CH₃)₂ CH₂ C(CH₃)₂ CH₂- oder
- C(CH₃)₂ CH₂ CH(CH₃) CH₂ - oder
für ein Phenylen, insbesondere für den bivalenten Rest der Formel

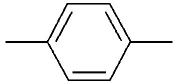

Bei m = 3 steht A bevorzugt für den trivalenten Rest der Formel

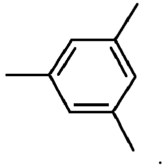

Bevorzugt sind solche Verbindungen der Formel (V), die den Formeln (Va) - (Vd) entsprechen.

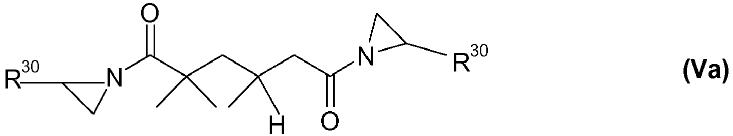

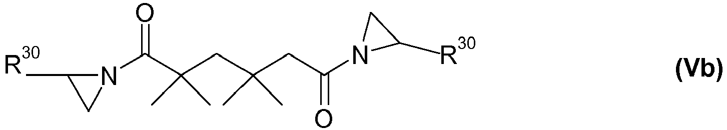

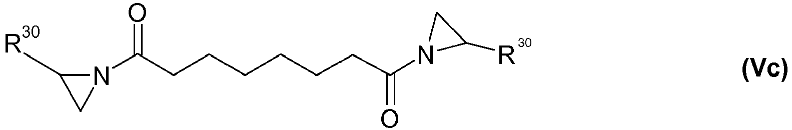

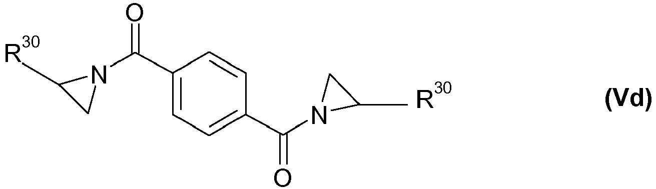

Ebenfalls bevorzugt sind als polyfunktionelle Aziridinverbindungen, Michael-Additionsprodukte von gegebenenfalls substituiertem Ethylenimin an Ester von mehrwertigen Alkoholen mit α,β-ungesättigten Carbonsäuren und den Additionsprodukten von gegebenenfalls substituiertem Ethylenimin an Polyisocyanate.

Geeignete Alkoholkomponenten sind beispielsweise Trimethylolpropan, Neopentylglykol, Glycerin, Pentaerythrit, 4,4'-Isopropylidendiphenol und 4,4'-Methylendiphenol. Als α,β-ungesättigte Carbonsäuren kommen bspw. Acryl- und Methacrylsäure, Crotonsäure und Zimtsäure in Frage.

Besonders bevorzugt enthält die erfindungsgemäße Zusammensetzung Acrylsäureester.

Die korrespondierenden mehrwertigen Alkohole der α,β-ungesättigten Carbonsäureester können ggf. Alkohole sein, die an ihren OH-Funktionen teilweise vollständig mit Alkylenoxiden einfach oder mehrfach verlängert sind. Hierbei kann es sich bspw. um die mit Alkylenoxiden einfach oder mehrfach verlängerten oben genannten Alkohole handeln. Diesbezüglich wird auch auf die US 4,605,698 verwiesen, dessen Offenbarung durch Bezugnahme in die vorliegende Erfindung eingeschlossen ist. Erfindungsgemäß besonders geeignete Alkylenoxide sind Ethylenoxid und Propylenoxid.

Beispiele für zur Reaktion mit gegebenenfalls substituiertem Ethylenimin geeigneten Polyisocyanaten sind die auf S. 4, Zeile 33 - 35 von WO2004/050617 A genannten.

Beispiele für erfindungsgemäß geeignete Aziridine sind die auf S. 3, Zeile 29 - 34 von WO2004/050617 A genannten.

Bevorzugt sind ebenfalls solche Aziridine wie sie beispielsweise in US 3,225,013 (Fram), US 4,490,505 (Pendergrass) und US 5,534,391 (Wang) beschrieben sind.

Ebenfalls bevorzugt sind solche Aziridine der Formel (I), die wenigsten drei Aziridingruppen besitzen, wie beispielsweise Trimethylolpropan-tris[3-(1-aziridinyl)propionat], Trimethylolpropan-tris[3-(2-methyl-1-aziridinyl)propionat], Trimethylolpropan-tris [2-aziridinylbutyrat], Tris(1-aziridinyl)phosphinoxid, Tris(2-methyl-1-aziridinyl)phosphinoxid, Pentaerythritol-tris-[3-(1-aziridinyl)propionat] und Pentaerythritol-tetrakis-[3-(1-aziridinyl)propionat].

Hiervon sind besonders Trimethylolpropan-tris[3-(1-aziridinyl)propionat], Trimethylolpropan-tris[3-(2-methyl-1-aziridinyl)propionat], Trimethylolpropan-tris [2-aziridinylbutyrat], Pentaerythritol-tris-[3-(1-aziridinyl)propionat] und Pentaerythritol-tetrakis-[3-(1-aziridinyl)propionat] bevorzugt.

Besonders sind Trimethylolpropan-tris[3-(1-aziridinyl)propionat], Trimethylolpropan-tris[3-(2-methyl-1-aziridinyl)propionat] und Pentaerythritol-tetrakis-[3-(1-aziridinyl)propionat] bevorzugt.

Ebenfalls bevorzugt sind polyfunktionelle Aziridine der Formel (VI) 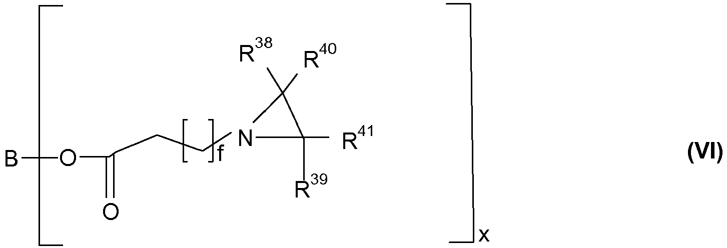 worin
- B: der Rest eines aliphatischen Polyols ist, das wenigstens x OH-Funktionen aufweist, wobei x OH-Funktionen durch den Rest der obigen Klammer substituiert sind,
- f: für eine Zahl von 0 bis 6, insbesondere von 1 bis 3 steht,
- x: eine Zahl größer oder gleich 2 ist, insbesondere für 2 bis 100.000 steht und
- R³⁸ und R³⁹ oder R⁴⁰ und R⁴¹: gemeinsam mit den Kohlenstoffatomen, an die sie gebunden sind, einen 5- bis 10 gliedrigen carbocyclischen Ring bilden, der unsubstituiert oder substituiert und/oder einfach- oder mehrfach ethylenisch ungesättigt ist.

Besonders bevorzugt ist B der Rest eines Poyvinylalkohols. Besonders bevorzugt sind solche Aziridine der Formel (VI), worin x für 3 oder 4 steht und B ein 3- oder 4-fach OH-funktionelles Polyol ist.

Besonders bevorzugt sind Aziridine der Formel (VI), die der Formel (VIa) - (VIc) entsprechen 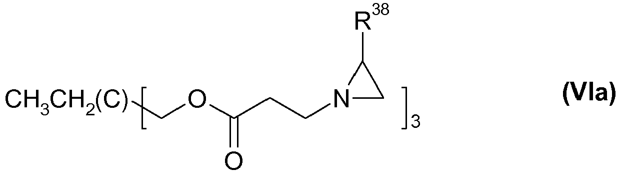 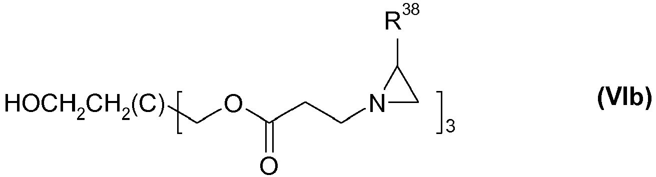 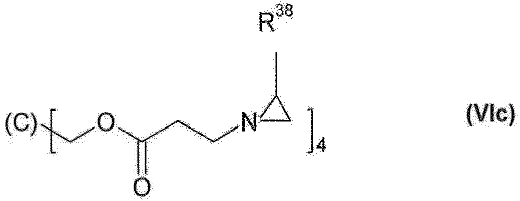 worin
R³⁸ für Wasserstoff oder CH₃ steht.

Besonders bevorzugt ist die auch als Crosslinker CX-100 von DSM bekannte Aziridinverbindung der Formel (VIa), mit R³⁸ = Methyl sowie auch das Produkt "Corial Härter AN" der BASF, das das Aziridin der Formel (VIa) mit R³⁸ = Wasserstoff enthält.
Die Stickstoff enthaltenden Polymere werden durch Umsetzung von Aziridinen, wie insbesondere den vorgenannten in Gegenwart von Wasser und gegebenenfalls in Gegenwart von Cosolventien erhalten. Die Stickstoff enthaltenden Polymere werden dadurch hergestellt, dass Aziridine in Gegenwart von Wasser und gegebenenfalls Cosolventien umgesetzt werden.

Die eingesetzte Wassermenge kann hierbei in einem breiten Bereich variiert werden. Im Allgemeinen setzt man mindestens 10 Gew. % Wasser bezogen auf die eingesetzten Aziridine ein. Bevorzugt beträgt die Wassermenge 20 bis 1,000 Gew. %, besonders bevorzugt 30 bis 300 Gew. % bezogen auf die eingesetzten Aziridine ein. Die einsetzbare Wassermenge ist nach oben prinzipiell nicht begrenzt, allerdings verursachen hohe Wassermengen naturgemäß einen höheren Isolierungsaufwand für die Stickstoff enthaltenden Polymere.

Die Reaktionstemperatur beträgt beispielsweise 30 bis 100 °C, bevorzugt 40 bis 90 °C und ganz bevorzugt 50 bis 80 °C.

Vorzugsweise wird die Umsetzung solange geführt, bis 95 % oder mehr, vorzugsweise 98 % oder mehr, besonders bevorzugt 99 % oder mehr des eingesetzten Aziridins bezogen auf den Anteil an Aziridinringen zur Umsetzung gebracht wurde. Ganz besonders bevorzugt wird die Reaktion so lange geführt bis keine Aziridinringe mehr nachweisbar sind.

Demzufolge weisen die erfindungsgemäß eingesetzten Stickstoff enthaltenden Polymere einen Anteil von 5 % oder weniger, vorzugsweise 2 % oder weniger, besonders bevorzugt 1 % oder weniger, ganz besonders bevorzugt keine nachweisbaren Gehalte an Aziridinringen bezogen auf die eingesetzten Aziridine auf.

In einer anderen Ausführungsform auf weisen die erfindungsgemäß eingesetzten Stickstoff enthaltenden Polymere einen Anteil von 5 % oder weniger, vorzugsweise 2 % oder weniger, besonders bevorzugt 1 % oder weniger, ganz besonders bevorzugt keine nachweisbaren Gehalte an Aziridin-Stickstoff bezogen auf den Gesamtstickstoffgehalt auf.

Der Anteil an unreagierten Aziridinringen kann beispielsweise mittels 13C-NMR Spektren im Vergleich zum eingesetzten Aziridin bestimmt werden.

Im Allgemeinen beträgt die Reaktionsdauer 2 bis 48 h, ganz bevorzugt 3 bis 24 h.

Der Einsatz von Cosolventien ist zwar zur Erreichung der gewünschten Stabilisatoren nicht zwingend erforderlich, kann aber besonders beim Einsatz hoher Aziridinkonzentrationen hilfreich sein, da eine Gelbildung im Reaktionsansatz auf diese Weise wirksam verhindert wird.

Als Cosolventien können im Allgemeinen alle Verbindungen eingesetzt werden, die mit Wasser mischbar sind und unter den Reaktionsbedingungen selbst nicht oder nur geringfügig mit den eingesetzten Aziridinen reagieren.

Bevorzugte Cosolventien sind Oligo- oder Polyalkylenglycole oder Triole, oder Ether der vorgenannten Verbindungen, insbesondere mit einem Molekulargewicht von kleiner als 1,000 g/mol. Besonders bevorzugt sind Ethylenglycol, Diethylenglycol, Triethylenglycol, Tetraethylenglycol, Polyethylenglycol, Propylenglycol, Dipropylenglycol, Polypropylenglcol, Glycerin sowie Mono-, DiMethyl-, Ethyl-, Propyl- oder Butylether der vorgenannten Verbindungen sowie beliebige Mischungen der vorgenannten Cosolventien.

Ganz besonders bevorzugt verwendet man Diethylenglycolbutylether als Cosolvenz.

Die eingesetzte Menge an Cosolventien kann in einem breiten Bereich variiert werden. Im Allgemeinen setzt man beispielsweise mindestens 20 Gew. % Cosolventien bezogen auf die eingesetzte Wassermenge ein, bevorzugt 30 bis 1,000 Gew. %, besonders bevorzugt 50 bis 300 Gew. %.

Die nach dem erfindungsgemäßen Verfahren hergestellten Stickstoff enthaltenden Polymere können direkt in Form der resultieren Lösung zur Stabilisierung Iod enthaltender Verbindungen eingesetzt werden oder nach Abtrennung von Cosolventien und/oder Wasser gegebenenfalls in isolierter Form.

Aufgrund ihrer stabilisierenden Wirkung eignen sich die Stickstoff enthaltenden Polymere wie insbesondere die erfindungsgemäß erhältlichen bzw. nach dem erfindungsgemäßen Verfahren hergestellten Stickstoff enthaltenden Polymere zur gemeinsamen Anwendung mit Iod enthaltenden Verbindungen in bioziden Mitteln.

Von der Erfindung sind daher auch biozide Mittel umfasst enthaltend zumindest
a) mindestens eine Iod enthaltende Verbindung mit biozider Wirkung
b) mindestens ein Stickstoff enthaltendes Polymer,
wobei die für die einzelnen Komponenten oben genannten bereiche und Vorzugsbereiche in gleicher Weise gelten.

Bevorzugte biozide Mittel enthalten
a) IPBC und
b) Stickstoff enthaltende Polymere erhältlich durch Umsetzung von zumindest einem bevorzugt genau einem Aziridin der Formel (VI) in Gegenwart von Wasser.

Die erfindungsgemäßen bioziden Mittel enthalten im Allgemeinen
a) 0,01 bis 70 Gew.-%, vorzugsweise 0,05 bis 60 Gew.-%, besonders bevorzugt 0,1 bis 50 Gew.-% an Iod enthaltenden Verbindungen mit biozider Wirkung und
b) 0,001 bis 50 Gew.-%, vorzugsweise 0,005 bis 40 Gew.-%, besonders bevorzugt 0,01 bis 30 Gew.-% an Stickstoff enthaltenden Polymeren.

Bevorzugt enthalten die erfindungsgemäßen bioziden Mittel die Iod enthaltenden Verbindungen mit biozider Wirkung, und die Stickstoff enthaltenden Polymeren in Summe von 0,011 bis 100 Gew.-%, bevorzugt 0,05 bis 80 Gew.-%, besonders bevorzugt 0,1 bis 60 Gew.-%.

In einer Ausführungsform enthalten die erfindungsgemäßen bioziden Mittel von 1 bis 280 Gew.-% an Stickstoff enthaltenden Polymeren, bevorzugt 2 bis 225 Gew.-%, insbesondere 5 bis 110 Gew.%, bezogen auf die Iod enthaltenden Verbindungen mit biozider Wirkung.

Die bioziden Mittel können weiterhin Lösungsmittel enthalten oder nicht.

Bei den gegebenenfalls enthaltenen Lösungsmitteln können solche Lösungsmittel eingesetzt werden, wie sie bereits oben als Cosolventien für die Umsetzung zu Sickstoff enthaltenden Polymeren beschrieben wurden. Die Bereiche und Vorzugsbereiche gelten hier analog.

Die bioziden Mittel können weiterhin Säuren wie beispielsweise organische und/oder anorganische Säuren enthalten oder nicht.

Dem Fachmann ist klar dass aufgrund der möglichen Basizität der Stickstoff enthaltenden Polymeren, insbesondere wenn sie aus Aziridinen erhalten wurden, die Säuren zumindest nicht vollständig in freier Form im bioziden Mittel vorliegen. Die unten gemachten Mengenangaben beziehen sich daher auf Gehalte und Mengen jeweils berechnet auf die freie Säure.

Bei den gegebenenfalls enthaltenen anorganischen Säuren kann es sich prinzipiell um alle anorganischen Säuren handeln, die im bioziden Mittel löslich sind. Bevorzugte anorganische Säuren sind Salzsäure bzw. HCl, Schwefelsäure und Phosphorsäure.

Bei den gegebenenfalls enthaltenen organischen Säuren kann es sich prinzipiell um alle organischen Säuren handeln, die im bioziden Mittel löslich sind. Bevorzugte organische Säuren sind Ameisensäure, Essigsäure, Citronensäure, Propionsäure oder Benzoesäure. Besonders bevorzugt wird Ameisensäure.

Der Gehalt an Säuren kann in einem breiten Bereich variiert werden. Im Allgemeinen beträgt er 0,01 bis 2 Gew.-%, bevorzugt 0,03 bis 1,5 Gew.-% und ganz besonders bevorzugt 0,05 bis 1 Gew.-% bezogen auf das gesamte biozide Mittel.

Die oben beschriebenen bioziden Mittel können zusätzlich noch weitere Wirkstoffe und Hilfsstoffe enthalten. Sie können beispielsweise als Lösung, Emulsion oder Suspension vorliegen.

Es können beispielsweise organische Lösungsmittel enthalten sein oder nicht.

Organische Lösungsmittel sind beispielsweise Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, wie beispielsweise Erdölfraktionen (Testbenzin, Shellsol D60 der Fa. Shell Chemical), einwertige Alkohole wie beispielsweise Ethanol, Isopropanol und Butanol, mehrwertige Alkohole wie beispielsweise Glycerin, Pentaerythritol, Polyvinylalkohol (z.B. Mowiol^{®} der Fa. Kuraray), Glykole wie beispielsweise Ethylenglykol und Propylenglykol, Oligoglykole und Polyglykole, Ether von Oligoglykolen wie bspw. Dipropylenglykol-Monomethylether (z.B. Dowanol^{®} TPM der Fa. Dow), Ether und Ester von Alkoholen wie (Texanol^{®} der Fa. Eastman), Ketone, wie beispielsweise Aceton, Methylethylketon, Methylisobutylketon oder Cyclohexanon, stark polar aprotische Lösungsmittel, wie beispielsweise Dimethylformamid und Dimethylsulfoxid, sowie z.B. vollveretherte Glykole, Oligoglykole und Polyglykole wie beispielsweise Ethylenglykoldibutylether, veretherte Polyole und veresterte Polyole, Ester von ein- sowie mehrwertigen Carbonsäuren, z.B. Adipinsäurediisobutylester, Maleinsäurediisobutylester (z.B. Rhodiasolv DIB^{®}).

Weiterhin können die erfindungsgemäßen bioziden Mittel als weitere Inhaltsstoffe Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide sowie mineralische und vegetabile Öle enthalten oder jeweils nicht.

Weiterhin können die erfindungsgemäßen bioziden Mittel als weitere Inhaltsstoffe Farbstoffe wie anorganische Pigmente, z.B. Eisenoxid, Titanoxid, Ferrocyanblau und organische Farbstoffe, wie Alizarin-, Azo- und Metallphthalocyaninfarbstoffe enthalten.

Weiterhin können die erfindungsgemäßen bioziden Mittel noch weitere Stabilisatoren enthalten, wie beispielsweise Chelatisierungsreagentien oder organische Epoxide. In vielen Fällen werden hier synergistische Effekte beobachtet.

Die Wirksamkeit und das Wirkungsspektrum der erfindungsgemäßen bioziden Mittel kann erhöht werden, wenn sie gegebenenfalls weitere Wirkstoffe ausgewählt aus der Gruppe der weitere antimikrobiell wirksame Verbindungen, Fungizide, Bakterizide, Herbizide, Insektizide oder andere Wirkstoffe enthalten sind.

In vielen Fällen erhält man dabei synergistische Effekte, d.h. die Wirksamkeit der Mischung ist größer als die Wirksamkeit der Einzelkomponenten. Besonders günstige Mischungspartner sind z.B. die folgenden Verbindungen, die jeweils einzeln für sich jeweils enthalten sein können oder nicht:
Triazole wie: Azaconazol, Azocyclotin, Bitertanol, Bromuconazol, Cyproconazol, Diclobutrazol, Difenoconazol, Diniconazol, Epoxyconazol, Etaconazol, Fenbuconazol, Fenchlorazol, Fenethanil, Fluquinconazol, Flusilazol, Flutriafol, Furconazol, Hexaconazol, Imibenconazol, Ipconazol, Isozofos, Myclobutanil, Metconazol, Paclobutrazol, Penconazol, Propioconazol, Prothioconazol, Simeoconazol, (±)-cis-1-(4-chlorphenyl)-2-(1H-1,2,4-triazol-1-yl)-cycloheptanol, 2-(1-tert-Butyl)-1-(2-chlorphenyl)-3-(1,2,4-triazol-1-yl)-propan-2-ol, Tebuconazol, Tetraconazol, Triadimefon, Triadimenol, Triapenthenol, Triflumizol, Triticonazol, Uniconazol sowie deren Metallsalze und Säureaddukte;
Imidazole wie:
   Clotrimazol, Bifonazol, Climbazol, Econazol, Fenapamil, Imazalil, Isoconazol, Ketoconazol, Lombazol, Miconazol, Pefurazoat, Prochloraz, Triflumizol, Thiazolcar 1-Imidazolyl-1-(4'-chlorophenoxy)-3,3-dimethylbutan-2-on sowie deren Metallsalze und Säureaddukte;
Pyridine und Pyrimidine wie:
   Ancymidol, Buthiobat, Fenarimol, Mepanipyrin, Nuarimol, Pyroxyfur, Triamirol;
Succinat-Dehydrogenase Inhibitoren wie:
   Benodanil, Carboxim, Carboximsulfoxid, Cyclafluramid, Fenfuram, Flutanil, Furcarbanil, Furmecyclox, Mebenil, Mepronil, Methfuroxam, Metsulfovax, Nicobifen, Pyrocarbolid, Oxycarboxin, Shirlan, Seedvax;
Naphthalin-Derivate wie:
   Terbinafin, Naftifin, Butenafin, 3-Chloro-7-(2-aza-2,7,7-trimethyl-oct-3-en-5-in);
Sulfenamide wie:
   Dichlofluanid, Tolylfluanid, Folpet, Fluorfolpet; Captan, Captofol;
Benzimidazole wie:
   Carbendazim, Benomyl, Fuberidazole, Thiabendazol oder deren Salze;
Morpholinderivate wie:
   Aldimorph, Dimethomorph, Dodemorph, Falimorph, Fenpropidin, Fenpropimorph, Tridemorph, Trimorphamid und ihre arylsulfonsauren Salze, wie z.B. p-Toluolsulfonsäure und p-Dodecylphenylsulfonsäure;
Benzthiazole wie:
   2-Mercaptobenzothiazol;
Benzthiophendioxide wie:
   Benzo[b]thiophen-S,S-dioxid-carbonsäurecyclohexylamid;
Benzamide wie:
   2,6-Dichloro-N-(4-trifluoromethylbenzyl)-benzamid, Tecloftalam;
Borverbindungen wie:
   Borsäure, Borsäureester, Borax;
Formaldehyd und Formaldehydabspaltende Verbindungen wie:
   Benzylalkoholmono-(poly)-hemiformal, 1,3-Bis(hydroxymethyl)-5,5-dimethylimidazolidine-2,4-dione (DMDMH), Bisoxazolidine, n-Butanol-hemiformal, Cis 1-(3-Chlorallyl)-3,5,7-triaza-1-azoniaadamantane chlorid, 1-[1,3-Bis(hydroxymethyl-2,5-dioxoimidazolidin-4-yl]-1,3-bis(hydroxymethyl)urea, Dazomet, Dimethylolharnstoff, 4,4-Dimethyl-oxazolidine, Ethylenglycolhemiformal, 7-Ethylbicyclooxazolidine, Hexa-hydro-S-triazine, Hexamethylentetramin, N-Hydroxymethyl-N'-methylthioharnstoff, Methylenbismorpholin, Natrium N-(Hydroxymethyl)glycinat, N-Methylolchloracetamid, Oxazolidine, Paraformaldehyd, Taurolin, Tetrahydro-1,3-oxazin, N-(2-Hydroxypropyl)-amin-methanol, Tetramethylol-acetylen-diharnstoff (TMAD);
Isothiazolinone wie:
   N-Methylisothiazolin-3-on, 5-Chlor-N-methylisothiazolin-3-on, 4,5-Dichloro-N-octylisothiazolin-3-on, 5-Chlor-N-octylisothiazolinon, N-Octyl-isothiazolin-3-on, 4,5-Trimethylen-isothiazolinon, 4,5-B enzis othiazolinon;
Aldehyde wie:
   Zimtaldehyd, Formaldehyd, Glutardialdehyd, ß-Bromzimtaldehyd, o-Phthaldialdehyd;
Thiocyanate wie:
   Thiocyanatomethylthiobenzothiazol, Methylenbisthiocyanat;
quartäre Ammoniumverbindungen und Guanidine wie:
   Benzalkoniumchlorid, Benzyldimethyltetradecylammoniumchlorid, Benzyldimethyldodecylammoniumchlorid, Dichlorbenzyldimethylalkylammoniumchlorid, Didecyldimethylammoniumchlorid, Dioctyldimethylammoniumchlorid, N-Hexadecyltrimethylammoniumchlorid, 1-Hexadecylpyridiniumchlorid, Iminoctadine-tris(albesilat);
Phenole wie:
   Tribromphenol, Tetrachlorphenol, 3-Methyl-4-chlorphenol, 3,5-Dimethyl-4-chlorphenol, Dichlorphen, 2-Benzyl-4-chlorphenol, Triclosan, Diclosan, Hexachlorophen, p-Hydroxybenzoesäuremethylester, p-Hydroxybenzoesäureethylester, p-Hydroxybenzoesäurepropylester, p-Hydroxybenzoesäurebutylester, p-Hydroxybenzoesäureoctylester,o-Phenylphenol, m-Phenylphenol, p-Phenylphenol, 4-(2-tert.-Butyl-4-methyl-phenoxy)-phenol, 4-(2-Isopropyl-4-methyl-phenoxy)-phenol, 4-(2,4-Dimethyl-phenoxy)-phenol und deren Alkali- und Erdalkalimetallsalze;
Mikrobizide mit aktivierter Halogengruppe wie:
   Bronopol, Bronidox, 2-Brom-2-nitro-1,3-propandiol, 2-Brom-4'-hydroxy-acetophenon, 1-Brom-3-chlor-4,4,5,5-tetramethyl-2-imidazoldinone, ß-Brom-ß-nitrostyrol, Chloracetamid, Chloramin T, 1,3-Dibrom-4,4,5,5-tetrametyl-2-imidazoldinone, Dichloramin T, 3,4-Dichlor-(3H)-1,2-dithiol-3-on, 2,2-Dibrom-3-nitril-propionamid, 1,2-Dibrom-2,4-dicyanobutan, Halane, Halazone, Mucochlorsäure, Phenyl-(2-chlor-cyan-vinyl)sulfon, Phenyl-(1,2-dichlor-2-cyanvinyl)sulfon, Trichlorisocyanursäure;
Pyridine wie:
   1-Hydroxy-2-pyridinthion (und ihre Cu-, Na-, Fe-, M n-, Zn-Salze), Tetrachlor-4-methylsulfonylpyridin, Pyrimethanol, Mepanipyrim, Dipyrithion, 1-Hydroxy-4-methyl-6-(2,4,4-trimethylpentyl)-2(1H)-pyridin;
Methoxyacrylate oder Ähnliche wie:
   Azoxystrobin, Dimoxystrobin, Fluoxastrobin, Kresoxim-methyl, Metominostrobin, Orysastrobin, Picoxystrobin, Pyraclostrobin, Trifloxystrobin, 2,4-dihydro-5-methoxy-2-methyl-4-[2-[[[[1-[3-(trifluoromethyl)phenyl]ethylidene]amino]oxy]methyl]phenyl]-3H-1,2,4-triazol-3-on e (CAS-Nr. 185336-79-2);
Metallseifen wie:
   Salze der Metalle Zinn, Kupfer und Zink mit höheren Fett-, Harz-, Naphthensäuren und Phosphorsäure wie z.B. Zinn-, Kupfer-, Zinknaphtenat, -octoat, 2-ethylhexanoat, -oleat, -phosphat, -benzoat;
Metallsalze wie:
   Salze der Metalle Zinn, Kupfer, Zink, sowie auch Chromate und Dichromate wie z.B. Kupferhydroxycarbonat, Natriumdichromat, Kaliumdichromat, Kaliumchromat, Kupfersulfat, Kupferchlorid, Kupferborat, Zinkfluorosilikat, Kupferfluorosilikat;
Oxide wie:
   Oxide der Metalle Zinn, Kupfer und Zink wie z.B. Tributylzinnoxid, Cu₂O, CuO, ZnO;
Oxidationsmittel wie:
   Wasserstoffperoxid, Peressigsäure, Kalium-persulfat;
Dithiocarbamate wie:
   Cufraneb, Ferban, Kalium-N-hydroxymethyl-N'-methyl-dithiobarbamat, Na- oder K-dimethyldithiocarbamat, Macozeb, Maneb, Metam, Metiram, Thiram, Zineb, Ziram;
Nitrile wie:
   2,4,5,6-Tetrachlorisophthalodinitril, Dinatrium-cyano-dithioimidocarbamat;
Chinoline wie:
   8-Hydroxychinolin und deren Cu-Salze;
sonstige Fungizide und Bakterizide wie:
   Bethoxazin, 5-Hydroxy-2(5H)-furanon; 4,5-Benzdithiazolinon, 4,5-Trimethylendithiazolinon, N-(2-p-Chlorbenzoylethyl)-hexaminiumchlorid, 2-Oxo-2-(4-hydroxy-phenyl)acethydroximsäure-chlorid, Tris-N-(cyclohexyldiazeniumdioxy)-aluminium, N-(Cyclo-hexyldiazeniumdioxy)-tributylzinn bzw. K-Salze, Bis-N-(cyclohexyldiazeniumdioxy)-kupfer, Iprovalicarb, Fenhexamid, Spiroxamin, Carpropamid, Diflumetorin, Quinoxyfen, Famoxadone, Polyoxorim, Acibenzolar-S-methyl, Furametpyr, Thifluzamide, Methalaxyl-M, Benthiavalicarb, Metrafenon, Cyflufenamid, Tiadinil, Teebaumöl, Phenoxyethanol,
   Ag, Zn oder Cu-haltige Zeolithe allein oder eingeschlossen in polymere Werkstoffe.

### Ganz besonders bevorzugt sind Mischungen mit

Azaconazol, Bromuconazol, Cyproconazol, Dichlobutrazol, Diniconazol, Diuron, Hexaconazol, Metaconazol, Penconazol, Propiconazol, Tebuconazol, Dichlofluanid, Tolylfluanid, Fluorfolpet, Methfuroxam, Carboxin, Benzo[b]thiophen-S,S-dioxid-carbonsäurecyclohexylamid, Fenpiclonil, 4-(2,2-Difluoro-1,3-benzodioxol-4-yl)-1H-pyrrol-3-carbonitril, Butenafin, Imazalil, N-Methyl-isothiazolin-3-on, 5-Chlor-N-methylisothiazolin-3-on, N-Octylisothiazolin-3-on, Dichlor-N-octylisozhiazolinon, Mercaptobenzthiazol, Thiocyanatomethylthiobenzothiazol, Thiabendazol, Benzisothiazolinon, N-(2-Hydroxypropyl)-amino-methanol, Benzylalkohol-(hemi)-formal, N-Methylolchloraceta m i d, N-(2-Hydroxypropyl)-amin-methanol, Glutaraldehyd, Omadine, Zn-Omadine, Dimethyldicarbonat, 2-Brom-2-nitro-1,3-propandiol, Bethoxazin, o-Phthaldialdehyd, 2,2-Dibrom-3-nitril-propionamid, 1,2-Dibrom-2,4-dicyanobutan, 1,3-Bis(hydroxymethyl)-5,5-dimethylimidazolidine-2,4-dione (DMDMH), Tetramethylol-acetylen-diharnstoff (TMAD), , Ethylenglycol-hemiformal, p-Hydroxybenzoesäure, Carbendazim, Chlorophen, 3-Methyl-4-chlorphenol, o-Phenylphenol.

Desweiteren werden neben den oben genannten Fungiziden und Bakteriziden auch gut wirksame Mischungen mit anderen Wirkstoffen hergestellt:
Insektizide / Akarizide / Nematizide:
   Abamectin, Acephat, Acetamiprid, Acetoprole, Acrinathrin, Alanycarb, Aldicarb, Aldoxycarb, Aldrin, Allethrin, Alpha-cypermethrin, Amidoflumet, Amitraz, Avermectin, Azadirachtin, Azinphos A, Azinphos M, Azocyclotin,
   Bacillus thuringiensis, Barthrin, 4-Bromo-2(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, Bioresmethrin, Bioallethrin, Bistrifluron, Bromophos A, Bromophos M, Bufencarb, Buprofezin, Butathiophos, Butocarboxin, Butoxycarboxim,
   Cadusafos, Carbaryl, Carbofuran, Carbophenothion, Carbosulfan, Cartap, Chinomethionat, Cloethocarb, Chlordane, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimidamid, Chlorpicrin, Chlorpyrifos A, Chlorpyrifos M, Cis-Resmethrin, Clocythrin, Clothiazoben, Cypophenothrin Clofentezin, Coumaphos, Cyanophos, Cycloprothrin, Cyfluthrin, Cyhalothrin, Cyhexatin, Cypermethrin, Cyromazin,
   Decamethrin, Deltamethrin, Demeton M, Demeton S, Demeton-S-methyl, Diafenthiuron, Dialiphos, Diazinon, 1,2-Dibenzoyl-1(1,1-dimethyl)-hydrazin, DNOC, Dichlofenthion, Dichlorvos, Dicliphos, Dicrotophos, Difethialon, Diflubenzuron, Dimethoat, 3,5-Dimethylphenyl-methylcarbamat, Dimethyl-(phenyl)-silyl-methyl-3-phenoxybenzylether, Dimethyl-(4-Ethoxyphenyl)-silylmethyl-3-phenoxybenzylether, Dimethylvinphos, Dioxathion, Disulfoton,
   Eflusilanate, Emamectin, Empenthrin, Endosulfan, EPN, Esfenvalerat, Ethiofencarb, Ethion, Ethofenprox, Etrimphos, Etoxazole, Etobenzanid,
   Fenamiphos, Fenazaquin, Fenbutatinoxid, Fenfluthrin, Fenitrothion, Fenobucarb, Fenothiocarb, Fenoxycarb, Fenpropathrin, Fenpyrad, Fenpyroximat, Fensulfothion, Fenthion, Fenvalerate, Fipronil, Flonicamid, Fluacrypyrim, Fluazuron, Flucycloxuron, Flucythrinate, Flufenerim, Flufenoxuron, Flupyrazofos, Flufenzine, Flumethrin Flufenprox, Fluvalinate, Fonophos, Formethanate, Formothion, Fosmethilan Fosthiazat, Fubfenprox, Furathiocarb,
   Halofenocid, HCH (CAS RN: 58-89-9), Heptenophos, Hexaflumuron, Hexythiazox, Hydramethylnon, Hydropren,
   Imidacloprid, Imiprothrin, Indoxycarb, Iprinomectin, Iprobenfos, Isazophos, Isoamidophos, Isofenphos, Isoprocarb, Isoprothiolane, Isoxathion, Ivermectin,
   Kadedrin
   Lambda-Cyhalothrin, Lufenuron,
   Malathion, Mecarbam, Mervinphos, Mesulfenphos, Metaldehyd, Methacrifos, Methamidophos, Methidathion, Methiocarb, Methomyl, Metalcarb, Milbemectin, Monocrotophos, Moxiectin,
   Naled, NI 125, Nicotin, Nitenpyram, Noviflumuron,
   Omethoat, Oxamyl, Oxydemethon M, Oxydeprofos,
   Parathion A, Parathion M, Penfluron, Permethrin, 2-(4-Phenoxyphenoxy)-ethyl-ethylcarbamat, Phenthoat, Phorat, Phosalon, Phosmet, Phosphamidon, Phoxim, Pirimicarb, Pirimiphos M, Pirimiphos A, Prallethrin, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyridalyl, Pyrimidifen, Pyriproxifen, Pyrithiobac-natrium
   Quinalphos,
   Resmethrin, Rotenon,
   Salithion, Sebufos, Silafluofen, Spinosad, Spirodiclofen, Spiromesifen, Sulfotep, Sulprofos,
   Tau-fluvalinat, Taroils, Tebufenozide, Tebufenpyrad, Tebupirimphos, Teflubenzuron, Tefluthrin, Temephos, Terbam, Terbufos, Tetrachlorvinphos, Tetramethrin, Tetramethacarb, Thiacloprid, Thiafenox, Thiamethoxam, Thiapronil, Thiodicarb, Thiofanox, Thiazophos, Thiocyclam, Thiomethon, Thionazin, Thuringiensin, Tralomethrin, Transfluthrin, Triarathen, Triazophos, Triazamate, Triazuron, Trichlorfon, Triflumuron, Trimethacarb,
   Vamidothion, Xylylcarb, Zetamethrin;
Molluscizide:
   Fentinacetat, Metaldehyd, Methiocarb, Niclosamid;
Herbizide und Algizide:
   Acetochlor, Acifluorfen, Aclonifen, Acrolein, Alachlor, Alloxydim, Ametryn, Amidosulfuron, Amitrole, Ammonium sulfamate, Anilofos, Asulam, Atrazine, Azafenidin, Aziptrotryn, Azimsulfuron,
   Benazolin, Benfluralin, Benfuresat, Bensulfuron, Bensulfid, Bentazon, Benzofencap, Benzthiazuron, Bifenox, Bispyribac, Bispyribac-Natrium, Borax, Bromacil, Bromobutide, Bromofenoxim, Bromoxynil, Butachlor, Butamifos, Butralin, Butylat, Bialaphos, Benzoyl-prop, Bromobutide, Butroxydim,
   Carbetamid, Carfentrazone-ethyl, Carfenstrol, Chlomethoxyfen, Chloramben, Chlorbromuron, Chlorflurenol, Chloridazon, Chlorimuron, Chlornitrofen, Chloressigsäure, Chloransulam-methyl, Cinidon-ethyl, Chlorotoluron, Chloroxuron, Chlorpropham, Chlorsulfuron, Chlorthal, Chlorthiamid, Cinmethylin, Cinofulsuron, Clefoxydim, Clethodim, Clomazone, Chlomeprop, Clopyralid, Cyanamide, Cyanazine, Cycloat, Cycloxydim, Chloroxynil, Clodinafop-propargyl, Cumyluron, Clometoxyfen, Cyhalofop, Cyhalofop-butyl, Clopyrasuluron, Cyclosulfamuron,
   Diclosulam, Dichlorprop, Dichlorprop-P, Diclofop, Diethatyl, Difenoxuron, Difenzoquat, Diflufenican, Diflufenzopyr, Dimefuron, Dimepiperate, Dimethachlor, Dimethipin, Dinitramine, Dinoseb, Dinoseb Acetate, Dinoterb, Diphenamid, Dipropetryn, Diquat, Dithiopyr, Diduron, DNOC, DSMA, 2,4-D, Daimuron, Dalapon, Dazomet, 2,4-DB, Desmedipham, Desmetryn, Dicamba, Dichlobenil, Dimethamid, Dithiopyr, Dimethametryn,
   Eglinazin, Endothal, EPTC, Esprocarb, Ethalfluralin, Ethidimuron, Ethofumesat, Ethobenzanid, Ethoxyfen, Ethametsulfuron, Ethoxysulfuron,
   Fenoxaprop, Fenoxaprop-P, Fenuron, Flamprop, Flamprop-M, Flazasulfuron, Fluazifop, Fluazifop-P, Fuenachlor, Fluchloralin, Flufenacet Flumeturon, Fluorocglycofen, Fluoronitrofen, Flupropanate, Flurenol, Fluridone, Flurochloridone, Fluroxypyr, Fomesafen, Fosamine, Fosametine, Flamprop-isopropyl, Flamprop-isopropyl-L, Flufenpyr, Flumiclorac-pentyl, Flumipropyn, Flumioxzim, Flurtamon, Flumioxzim, Flupyrsulfuron-methyl, Fluthiacet-methyl,
   Glyphosate, Glufosinate-ammonium
   Haloxyfop, Hexazinon,
   Imazamethabenz, Isoproturon, Isoxaben, Isoxapyrifop, Imazapyr, Imazaquin, Imazethapyr, Ioxynil, Isopropalin, Imazosulfuron, Imazomox, Isoxaflutole, Imazapic,
   Ketospiradox,
   Lactofen, Lenacil, Linuron,
   MCPA, MCPA-hydrazid, MCPA-thioethyl, MCPB, Mecoprop, Mecoprop-P, Mefenacet, Mefluidid, Mesosulfuron, Metam, Metamifop, Metamitron, Metazachlor, Methabenzthiazuron, Methazol, Methoroptryne, Methyldymron, Methylisothiocyanat, Metobromuron, Metoxuron, Metribuzin, Metsulfuron, Molinat, Monalid, Monolinuron, MSMA, Metolachlor, Metosulam, Metobenzuron,
   Naproanilid, Napropamid, Naptalam, Neburon, Nicosulfuron, Norflurazon, Natriumchlorat,
   Oxadiazon, Oxyfluorfen, Oxysulfuron, Orbencarb, Oryzalin, Oxadiargyl,
   Propyzamid, Prosulfocarb, Pyrazolate, Pyrazolsulfuron, Pyrazoxyfen, Pyribenzoxim, Pyributicarb, Pyridat, Paraquat, Pebulat, Pendimethalin, Pentachlorophenol, Pentoxazon, Pentanochlor, Petroleumöle, Phenmedipham, Picloram, Piperophos, Pretilachlor, Primisulfuron, Prodiamine, Profoxydim, Prometryn, Propachlor, Propanil, Propaquizafob, Propazine, Propham, Propisochlor, Pyriminobac-methyl, Pelargonsäure, Pyrithiobac, Pyraflufen-ethyl,
   Quinmerac, Quinocloamine, Quizalofop, Quizalofop-P, Quinchlorac,
   Rimsulfuron
   Sethoxydim, Sifuron, Simazine, Simetryn, Sulfosulfuron, Sulfometuron, Sulfentrazone, Sulcotrione, Sulfosate,
   Teeröle, TCA, TCA-Natrium, Tebutam, Tebuthiuron, Terbacil, Terbumeton, Terbutylazine, Terbutryn, Thiazafluoron, Thifensulfuron, Thiobencarb, Thiocarbazil, Tralkoxydim, Triallate, Triasulfuron, Tribenuron, Triclopyr, Tridiphane, Trietazine, Trifluralin, Tycor, Thdiazimin, Thiazopyr, Triflusulfuron,
   Vernolat.

Die bioziden Mittel eignen sich insbesondere zur bioziden Ausrüstung von technischen Materialien wie insbesondere Anstrichmitteln wie beispielsweise Farben, Lacken, Grundierungen, Imprägnierungen, Lasuren.

Besonders in Alkydharz enthaltenden Bindemittelformulierungen, insbesondere wenn sie Übergangsmetalltrockner und/oder Übergangsmetallverbindungen als Pigmente enthalten, kommt der stabilisierende Effekt der Stickstoff enthaltenden Polymere auf die Iod enthaltenden Verbindungen mit biozider Wirkung besonders vorteilhaft zum Tragen.

Die Erfindung betrifft daher weiterhin Bindemittelformulierungen, enthaltend
a) zumindest ein Bindemittel,
b) zumindest eine Iod enthaltende Verbindung mit biozider Wirkung und
c) zumindest ein Stickstoff enthaltendes Polymer
wobei die für die einzelnen Komponenten oben genannten Bereiche und Vorzugsbereiche in gleicher Weise gelten.

Bevorzugte Bindemittel sind oxidativ trocknende Bindemittel, wie beispielsweise Alkydharz enthaltende Bindemittel oder durch Koaleszenzmittel verfilmende Bindemittel wie insbesondere Polymerlatices.

Bei den Alkydharzen handelt es sich im Allgemeinen um Polykondensationsharze aus Polyolen und mehrwertigen Carbonsäuren bzw. deren Anhydriden und Fetten, Ölen oder freien natürlichen und/oder synthetischen Fettsäuren. Die Alkydharze können gegebenenfalls noch mit hydrophilen, insbesondere wasserlöslichen Gruppen chemisch modifiziert sein, um beispielsweise als emulgierbares bzw. als wasserlösliches Alkydharz einsetzbar zu sein.

Bevorzugt handelt es sich bei den genannten Polyolen um Glycerin, Pentaerythrit, Trimethylolethan, Trimethylolpropan sowie um verschiedene Diole wie Ethan-/Propandiol, Diethylenglycol und Neopentylglycol.

Bevorzugt handelt es sich bei den genannten mehrwertigen Carbonsäuren bzw. deren Anhydriden um Phthalsäure, Phthalsäureanhydrid, Maleinsäureanhydrid, Isophthalsäure, Terephthalsäure, Trimellitsäureanhydrid, Adipinsäure, Azelainsäure oder Sebacainsäure.

Bei den genannten Ölen oder Fettsäuren handelt es sich im allgemeinen um Leinöl, Oiticiaöl, Holzöl, Sojaöl, Sonnenblumenöl, Safloröl, Ricinenöl, Tallöl, Rizinusöl, Kokosöl, Erdnussöl, deren Fettsäuren sowie synthetischen Monocarbonsäuren.

Die Alkydharze können gegebenenfalls noch modifiziert werden bspw. mit Naturharzen, Phenolharzen, Acrylharzen, Styrol, Epoxidharzen, Siliconharzen, Isocyanaten, Polyamiden oder Aluminiumalkoholaten.

Die Alkydharze haben im allgemeinen eine Molmasse von 500 bis 100,000 g/mol, vorzugsweise von 1,000 bis 50,000 g/mol, insbesondere von 1,500 bis 20,000 g/mol, vorzugsweise bestimmt durch Laserlicht-Streuung, siehe bspw. "Static Light Scattering of Polystyrene Reference Materials: Round Robin Test", U.Just, B.Werthmann International Journal of Polymer Analysis and Characterization, 1999 Vol.5, Seiten 195 - 207.

Die erfindungsgemäßen Bindemittelformulierungen enthalten vorzugsweise 1 bis 80 Gew.-%, bevorzugt 2 bis 70 Gew.-% und besonders bevorzugt 3 bis 60 Gew.-% an Bindemittel, vorzugsweise Alkydharz.

Bevorzugt enthält die erfindungsgemäße Bindemittelformulierung weiterhin zumindest einen Übergangsmetalltrockner. Unter Übergangsmetalltrockner werden im Rahmen dieser Anmeldung insbesondere Übergangsmetallverbindungen verstanden, die die Trocknung und Härtung von Alkydharz enthaltenden Bindemitteln ermöglichen oder beschleunigen.

Bevorzugt sind die Salze von Übergangsmetallen der Gruppen Vb, VIb, VIIb, VIII und Ib des chemischen Periodensystems. Insbesondere handelt es sich um die Salze von Cobalt, Mangan, Vanadium, Nickel, Kupfer und Eisen, besonders bevorzugt um Cobalt, Mangan, Eisen und Vanadium. Sie müssen nicht unbedingt nur allein eingesetzt, sondern können auch in Kombination mit nicht Übergangsmetallsalzen, wie Beispielsweise Blei, Calcium oder Zirkonium zum Einsatz kommen.

Die bevorzugten Übergangsmetallsalze sind in Testbenzin, bei 20 °C in einer Menge von mehr als 10 g/l löslich. Vorzugsweise handelt es sich um die Salze von Carbonsäuren, die eine gute Verträglichkeit mit den Alkydharzen haben und gleichzeitig eine ausreichende Löslichkeit des Metallsalzes gewährleisten. Verwendet werden bevorzugt Übergangsmetallsalze von Fettsäuren wie Oleate oder Linoleate, Harzsäuren wie Resinate oder Salze der 2-Ethylhexansäure (Octoate). Bevorzugte Übergangsmetalltrockner sind Cobaltoctoat und Cobaltnaphthenat z.B. Octasoligen^{®}-Cobalt 12 der Firma Borchers.

Bevorzugt enthalten die erfindungsgemäßen Bindemittelformulierungen die Übergangsmetalltrockner in einer Menge von 0,001 bis 1 Gew.-%, bevorzugt 0,005 bis 0,5 Gew.-% und ganz besonders bevorzugt 0,01 bis 0,1 Gew.-%, jeweils bezogen auf das Bindemittel, vorzugsweise Alkydharz.

Die Bindemittelformulierungen enthalten in einer bevorzugten Ausführungsform wenigstens ein polares organisches Lösungsmittel, vorzugsweise ein polares protisches organisches Lösungsmittel. Beispielsweise enthalten sind polare protische organische Lösung wie Dipropylenglykol-monomethylether (z.B. Dowanol DPM der Fa. Dow Chemical) sowie weiterhin alternativ, vorzugsweise zusätzlich dazu polare aprotische organische Lösungsmittel, wie Dimethylformamid und Dimethylsulfoxid, sowie z.B. veretherte Glykole, Oligoglykole und Polyglykole, veretherte Polyole und veresterte Polyole, Ester von ein- sowie mehrwertigen Carbonsäuren, z.B. Adipinsäurediisobutylester, Maleinsäurediisobutylester (z.B. Rhodiasolv DIB ).

Besonders bevorzugte ist eine Bindemittelformulierung, enthaltend
1 bis 80 Gew.-%, bevorzugt 2 bis 70 Gew.-%, besonders bevorzugt 3 bis 60 Gew.-% Alkydharz
0 bis 50 Gew.-%, bevorzugt 0 bis 45 Gew.-%, besonders bevorzugt 0 bis 40 Gew.-%. Pigmente
0,01 bis 5 Gew.-%, bevorzugt 0,05 bis 3 Gew.-%, besonders bevorzugt 0,1 bis 2 Gew.-% Iod enthaltende Verbindungen mit biozider Wirkung, vorzugsweise IPBC
0,001 bis 5 Gew.-%, bevorzugt 0,005 bis 3 Gew.-%, besonders bevorzugt 0,01 bis 2 Gew.-% Stickstoff enthaltendes Polymer,
2 bis 97 Gew.-% Lösungsmittel, vorzugsweise solche wie sie oben für die bioziden Mittel beschrieben wurden und 0,001 bis 3 Gew.-% eines Übergangsmetalltrockners.

Die Bindemittelformulierung kann darüber hinaus noch jeweils unabhängig voneinander Füller, Hautverhinderungsmittel, Rheologieadditive wie beispielsweise Antiabsetzmittel und Thixotropiermittel, weitere antimikrobiell wirksame Verbindungen, Fungizide, Bakterizide, Herbizide, Insektizide oder andere Wirkstoffe enthalten oder nicht, wobei für die antimikrobiell wirksamen Verbindungen, Fungizide, Bakterizide, Herbizide, Insektizide oder andere Wirkstoffe die oben für die bioziden Mittel gemachten Angaben hier in gleicher Weise gelten.

Die Bindemittelformulierung kann darüber hinaus noch jeweils unabhängig voneinander Lösungsmittel, Prozessadditive, Weichmacher, Hitzestabilisatoren, sowie Korrosionsinhibitoren enthalten oder nicht.

Darüber hinaus können die erfindungsgemäßen bioziden Mittel oder Bindemittelformulierungen auch noch ein oder mehrere Hilfsmittel aus der Reihe der Antioxidantien, Radikalfänger, UV-Stabilisatoren, Chelatoren und UV-Absorber enthalten. Teilweise werden dabei synergistische Wirkungen beobachtet.

Beispielhaft seien als UV-Stabilisatoren genannt:
sterisch gehinderten Phenole, wie
2,6-Di-tert.-butyl-4-methylphenol, 2-tert.-Butyl-4,6-dimethylphenol, 2,6-Di-cyclopentyl-4-methylphenol, 2-(α-Methylcyclohexyl)-4,6-dimethylphenol, 2,6-Di-octadecyl-4-methylphenol oder 2,6-Di-tert.-butyl-4-methoxymethylphenol, Diethyl-(3,5-di-tert-butyl-4-hydroxybenzyl)phosphonate, 2,4-Dimethyl-6-(1-methylpentadecyl)-phenol, 2-Methyl-4,6-Bis[(octylthio)methyl]phenol, 2,6-Di-tert.-butyl- 4-methoxyphenol, 2,5-Di-tert.-butyl-hydrochinon, 2,5-Di-tert.-amyl-hydrochinon, 2,6-Diphenyl-4-octadecyloxyphenol, 2,2'-Thio-bis-(6-tert.-butyl-4-methylphenol), 2,2'-Thio-bis-(4-octylphenol), 4,4'-Thio-bis-(6-tert.-butyl-3-methylphenol), 4,4'-Thio-bis-(6-tert.-butyl-2-methylphenol), 2,2'-Methylen-bis-(6-tert.-butyl-4-methylphenol), 2,2'-Methylen-bis-(4-methyl-6-cyclohexylphenol), 2,2'-Methylen-bis-(4,6-di-tert.-butylphenol), 2,2'-Ethyliden-bis-(4,6-di-tert.-butylphenol), 4,4'-Methylen-bis-(2,6-di-tert.-butylphenol), 4,4'-Methylen-bis-(6-tert.-butyl- 2-methylphenol), 1,1-Bis-(5-tert.-butyl-4-hydroxy-2-methylphenyl)-butan, 1,1,3-Tris-(5-tert.-butyl-4-hydroxy-2-methylphenyl)-butan, 1,3,5-Tri-(3,5-di-tert.-butyl-4-hydroxybenzyl)-2,4,6-trimethylbenzol, 3,5-Di-tert.-butyl-4-hydroxybenzyl- mercaptoessigsäure-isooctylester, 1,3,5-Tris-(3,5-di-tert.-butyl-4-hydroxybenzyl)- isocyanurat, 1,3,5-Tris-(4-tert.butyl-3-hydroxy-2,6-dimethylbenzyl)-isocyanurat, 1,3,5-Tris[(3,5-di-tert-butyl-4-hydroxyphenyl)propionyloxyethyl] isocyanurate, 3,5-Di-tert.-butyl-4-hydroxybenzyl-phosphonsäure-dioctadecylester, 3,5-Di-tert.-butyl-4-hydroxybenzyl-phosphonsäure-monoethylester-Calciunr-salz, N,N'-Di-(3,5-di-tert.-butyl-4-hydroxyphenylpropionyl)-hexamethylendiamin, N,N'-Di-(3,5-di-tert.-butyl-4-hydroxyphenylpropionyl)-trimethylendiamin, N,N'-Di-(3,5-di-tert-butyl-4-hydroxyphenyl-propionyl)-hydrazin, 3,9-Bis[1,1-dimethyl-2-[(3-tert-butyl-4-hydroxy-5-methylphenyl)propionyloxy]ethyl]-2,4,8,10-tetraoxaspiro[5.5]undecane, Bis[3,3-bis(4'-hydroxy-3'-tert-butylphenyl)butanoic acid] ethylene glycol ester, 2,6-bis[[3-(1,1-dimethylethyl)-2-hydroxy-5-methylphenyl]octahydro-4,7-methano-1H-indenyl]-4-methyl-phenol (= Wingstay L), 2,4-Bis(n-octylthio)-6-(3,5-di-tert-butyl-4-hydroxyphenylamino)-s-triazine, N-(4-Hydroxyphenyl)octadecanamide, 2,4-Di-tert-butylphenyl 3',5'-di-tert-butyl-4'-hydroxybenzoate, (Benzoic acid, 3,5-bis(1,1-dimethylethyl)-4-hydroxy-, hexadecyl ester), 3-Hydroxyphenyl benzoate, 2,2'-Methylenebis(6-tert-butyl-4-methylphenol) monoacrylate, 2-(1,1-dimethylethyl)-6-[1-[3-(1,1-dimethylethyl)-5-(1,1-dimethylpropyl)-2-hydroxyphenyl]ethyl]-4-(1,1-dimethylpropyl)phenyl ester,
Ester der ß-(3,5-Di-tert.-butyl-4-hydroxyphenyl)-propionsäure mit ein- oder mehrwertigen Alkoholen wie z.B. mit Methanol, Octadecanol, 1,6-Hexandiol, Neopentylglycol, Thiodiethylenglycol, Diethylenglycol, Triethylenglycol, Pentaerythrit, Tris-hydroxyethyl-isocyanurat oder Di-hydroxyethyl-oxalsäurediamid,
Ester der ß-(5-tert--Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-hydroxyethyl-isocyanurat oder Di-hydroxyethyl-oxalsäurediamid.

Gehinderte Amine wie,
Bis(1,2,2,6,6-pentamethyl-4-piperidyl) 2-(3,5-di-tert-butyl-4-hydroxybenzyl)-2-butyl malonate, Bis(2,2,6,6-tetramethyl-4-piperidyl) decanedioate, Dimethyl succinate-1-(2-hydroxyethyl)-4-hydroxy-2,2,6,6-tetramethylpiperidine copolymer, Poly[[6-[(1,1,3,3-tetramethylbutyl)amino]-1,3,5-triazine-2,4-diyl][(2,2,6,6-tetramethyl-4-piperidyl)imino]hexamethylene[(2,2,6,6-tetramethyl-4-piperidyl)imino]] (CAS-Nr 71878-19-8), 1,5,8,12-Tetrakis[4,6-bis(N-butyl-N-1,2,2,6,6-pentamethyl-4-piperidylamino)-1,3,5-triazin-2-yl]-1,5,8,12-tetraazadodecane (CAS-Nr. 106990-43-6), Bis(1,2,2,6,6-pentamethyl-4-piperidyl) decanedioate, Bis(1,2,2,6,6-pentamethyl-4-piperidyl) 2-(3,5-di-tert-butyl-4-hydroxybenzyl)-2-butyl malonate, Decanedioic acid, bis(2,2,6,6-tetramethyl-4-piperidinyl) ester, reaction products with tert-Bu hydroperoxide and octane (CAS-Nr 129757-67-1), Chimasorb 2020 (CAS-Nr 192268-64-7), Poly[[6-morpholino-1,3,5-triazine-2,4-diyl][(2,2,6,6-tetramethyl-4-piperidinyl)imino]-1,6-hexanediyl[(2,2,6,6-tetramethyl-4-piperidinyl)imino]], Poly[[6-(4-morpholinyl)-1,3,5-triazine-2,4-diyl][(1,2,2,6,6-pentamethyl-4-piperidinyl)imino]-1,6-hexanediyl[(1,2,2,6,6-pentamethyl-4-piperidinyl)imino]] (9CI), 3-Dodecyl-1-(2,2,6,6-tetramethyl-4-piperidyl)pyrrolidine-2,5-dione, 3-Dodecyl-1-(1,2,2,6,6-pentamethylpiperidin-4-yl)pyrrolidine-2,5-dione"4-Octadecanoyloxy-2,2,6,6-tetramethylpiperidine, Poly[[6-(cyclohexylamino)-1,3,5-triazine-2,4-diyl][(2,2,6,6-tetramethyl-4-piperidinyl)imino]-1,6-hexanediyl[(2,2,6,6-tetramethyl-4-piperidinyl)imino]], 1H,4H,5H,8H-2,3a,4a,6,7a,8a-Hexaazacyclopenta[def]fluorene-4,8-dione, hexahydro-2,6-bis(2,2,6,6-tetramethyl-4-piperidinyl)- (CAS-Nr 109423-00-9), N,N'-Bis(formyl)-N,N'-bis(2,2,6,6-tetramethyl-4-piperidyl)-1,6-hexanediamine, N-(tetramethyl-4-piperidinyl)maleimide-C20-24-α-olefin copolymer (CAS-Nr 199237-39-3), Tetrakis(1,2,2,6,6-pentamethyl-4-piperidyl) 1,2,3,4-butanetetracarboxylate, Tetrakis(2,2,6,6-tetramethyl-4-piperidyl) 1,2,3,4-butanetetracarboxylate, 1,2,2,6,6-Pentamethyl-4-piperidinyltridecyl 1,2,3,4-butanetetracarboxylate, (1,2,3,4-Butanetetracarboxylic acid, 2,2,6,6-tetramethyl-4-piperidinyl tridecyl ester), (2,4,8,10-Tetraoxaspiro[5.5]undecane-3,9-diethanol, β,β,β',β'-tetramethyl-, polymer with 1,2,3,4-butanetetracarboxylic acid) (CAS-Nr 115055-30-6), 2,2,4,4-Tetramethyl-21-oxo-7-oxa-3,20-diazadispiro[5.1.11.2]heneicosane, (7-Oxa-3,20-diazadispiro[5.1.11.2]heneicosane-20-propanoic acid, 2,2,4,4-tetramethyl-21-oxo-, tetradecyl ester), (7-Oxa-3,20-diazadispiro[5.1.11.2]heneicosan-21-one, 2,2,4,4-tetramethyl-20-(oxiranylmethyl)-), (Propanamide, N-(2,2,6,6-tetramethyl-4-piperidinyl)-3-[(2,2,6,6-tetramethyl-4-piperidinyl)amino]-), (1,3-Propanediamine, N,N"-1,2-ethanediylbis-, polymer with 2,4,6-trichloro-1,3,5-triazine, reaction products with N-butyl-2,2,6,6-tetramethyl-4-piperidinamine) (CAS-Nr 136504-96-6), 1,1'-Ethylenebis(3,3,5,5-tetramethyl-2-piperazinone), (Piperazinone, 1,1',1"-[1,3,5-triazine-2,4,6-triyltris[(cyclohexylimino)-2,1-ethanediyl]]tris[3,3,5,5-tetramethyl-), (7-Oxa-3,20-diazadispiro[5.1.11.2]heneicosane-20-propanoic acid, 2,2,4,4-tetramethyl-21-oxo-, dodecyl ester), 1,1-Bis(1,2,2,6,6-pentamethyl-4-piperidyloxycarbonyl)-2-(4-methoxyphenyl)ethene, (2-Propenoic acid, 2-methyl-, methyl ester, polymer with 2,2,6,6-tetramethyl-4-piperidinyl 2-propenoate) (CAS-Nr 154636-12-1), (Propanamide, 2-methyl-N-(2,2,6,6-tetramethyl-4-piperidinyl)-2-[(2,2,6,6-tetramethyl-4-piperidinyl)amino]-), (D-Glucitol, 1,3:2,4-bis-O-(2,2,6,6-tetramethyl-4-piperidinylidene)-) (CA-Nr 99473-08-2), N,N'-Bis(2,2,6,6-tetramethyl-4-piperidinyl)isophthalamide, 4-Hydroxy-2,2,6,6-tetramethylpiperidine, 1-Allyl-4-hydroxy-2,2,6,6-tetramethylpiperidine, 1-Benzyl-4-hydroxy-2,2,6,6-tetramethylpiperidine, 1-(4-tert.-Butyl-2-butenyl)-4-hydroxy-2,2,6,6-tetramethylpiperidine, 4-Stearoyloxy-2,2,6,6-tetramethylpiperidine, 1-Ethyl-4-salicyloyloxy-2,2,6,6-tetramethylpiperidine, 4-Methacryloyloxy-1,2,2,6,6-pentamethylpiperidine, 1,2,2,6,6-Pentamethylpiperidin-4-yl-(3-(3,5-ditert.-butyl-4-hydroxyphenyl)-propionate, 1-Benzyl-2,2,6,6-tetramethyl-4-piperidinylmaleinate, (Di-2,2,6,6-tetramethylpiperidin-4-yl)-adipate, (Di-2,2,6,6-tetramethylpiperidin-4-yl)-sebacate, (Di-1,2,3,3,6-tetramethyl-2,6-diethyl-piperidin-4-yl)-sebacate, (Di-1-allyl-2,2,6,6-tetramethyl-piperidin-4-yl)phthalate, 1-Propargyl4-β-cyanoethyloxy-2,2,6,6-tetramethylpiperidine, 1-Acetyl-2,2,6,6-tetramethylpiperidin-4-yl-acetate, (Trimellitic acid-tri-(2,2,6,6-tetramethylpiperidin-4-yl)ester), 1-Acryloyl-4-benzyloxy-2,2,6,6-tetramethylpiperidine, Dibutyl-malonic acid-di-(1,2,2,6,6-pentamethyl-piperidin-4-yl)ester, Butyl-(3,5-di-tert-butyl-4-hydroxybenzyl)-malonic acid-di-(1,2,2,6,6-pentamethylpiperidin-4-yl)ester, Dibenzyl- malonic acid-di-(1,2,2,6,6-pentamethylpiperidin-4-yl)ester, Dibenzyl-malonic acid-di-(1,2,3,6-tetramethyl-2,6-diethyl-piperidin-4-yl)ester, Hexane-1',6'-bis-(4-carbamoyloxy-1-n-butyl-2,2,6,6-tetramethylpiperidine), Toluene-2',4'-bis-(4-carbamoyloxy-1-n-propyl-2,2,6,6-tetramethylpiperidine), Dimethyl-bis-(2,2,6,6- tetramethylpiperidine-4-oxy)silane, Phenyl-tris-(2,2,6,6-tetramethylpiperidine-4-oxy)silane, Tris-(-propyl-2,2,6,6-tetramethylpiperidin-4-yl)phosphite, Tris-(1-propyl-2,2,6,6-tetramethylpiperidin-4-yl)phosphate, Phenyl-[bis-(1,2,2,6,6-pentamethylpiperidin-4-yl)phosphonate, Di(1,2,2,6,6-pentamethylpiperidin- 4-yl)sebacate, NN'-Bis-(2,2,6,6-tetramethylpiperidin-4-yl)hexamethylene- 1,6-diamine, NN'-Bis-(2,2,6,6-tetramethylpiperidin-4-yl)hexamethylene- 1,6-diacetamide, 1-Acetyl-4-(N-cyclohexylacetamido)-2,2,6,6-tetramethylpiperidine, 4-Benzylamino-2,2,6,6-tetramethylpiperidine, N,N'-Bis-(2,2,6,6-tetramethylpiperidin-4-yl)-N,N'-dibutyladipamide, N,N'-Bis-(2,2,6,6-tetramethylpiperidin-4-yl)-N,N'-dicyclohexyl-(2-hydroxypropylene), NN'-Bis-(2,2,6,6-tetramethylpiperidin- 4-yl)-p-xylylenediamine, 4-(Bis-2-hydroxyethyl)-amino-1,2,2,6,6-pentamethylpiperidine, 4-(3-Methyl-4-hydroxy-5-tert.-butyl-benzoicacidamido)-2,2,6,6-tetramethylpiperidine, 4-Methacrylamino-1,2,2,6,6-pentamethylpiperidine, 9-Aza-8,8,10,10-tetramethyl-1,5-dioxaspiro[5.5]undecane, 9-Aza-8,8,10,10-tetramethyl-3-ethyl-1,5-dioxaspiro[5.51undecane, 8-Aza-2,7,7,8,9,9-hexamethyl- 1,4-dioxaspiro[4.5]decane, 9-Aza-3-hydroxymethyl-3-ethyl-8,8,9,10,10-pentamethyl-1-5-dioxaspiro[5.5]undecane, 9-Aza-3-ethyl-3-acetoxymethyl- 9-acetyl-8,8,10,10-tetramethyl- 1,5-dioxaspiro[5.5]undecane, 2,2,6,6-Tetramethylpiperidine-4-spiro-2'-(1',3'-dioxane)5'-spiro-5"-(1",3"-dioxane)-2"-spiro4"-(2"',2"',6"',6"'-tetramethylp iperidine),3-Benzyl-1,3,8-triaza-7,7,9,9-tetramethyl-spiro[4.5]decane-2,4-dione, 3-n-Octyl-1,3,8-triaza-7,7,9,9-tetramethyl-spiro[4.5]decane-2,4-dione, 3-Allyl-1,3,8-triaza-1,7,7,9,9-pentamethyl-spiro[4.5]decane-2,4.dione, 3-Glycidyl-1,3,8-triaza-7,7,8,9,9- pentamethyl-spiro(4.5]decane-2,4-dione, 2-Isopropyl-7,7,9,9-tetramethyl-1-oxa- 3,8-diaza-4-oxyspiro[4.5]decane, 2-Butyl-7,7,9,9-tetramethyl-1-oxa-3,8-diaza-4-oxyspiro[4.5]decane, 2-Isopropyl-7,7,9,9-tetramethyl-1-oxa-4,8-diaza-oxyspiro[4.5]decane, 2-Butyl-7,7,9,9-tetramethyl-1-oxa-4,8-diaza-3-oxyspiro[4.5]decane, Bis-[β-(2,2,6,6-tetramethylpiperidino)-ethyl]-sebacate, α-(2,2,6,6-tetramethylpiperidino)-acetic acid-n-octyl ester, 1,4-bis-(2,2,6,6-tetramethylpiperidino)- 2-butene, N-Hydroxymethyl-N'-2,2,6,6-tetramethylpiperidin-4-yl-urea, N-Methoxymethyl-N'-2,2,6,6-tetramethylpiperidin-4-yl-urea, N-Methoxymethyl-N'-n-dodecyl- N'-2,2,6,6-tetramethylpiperidin-4-yl-urea, O-(2,2,6,6-Tetramethylpiperidin-4-yl)- N-methoxymethyl-urethane.

Phosphite und Phosphonate wie,
Tri(nonylphenyl) phosphite, Tris(2,4-di-tert-butylphenyl) phosphite, Bis(2,4-di-tert-butylphenyl) pentaerythritol diphosphite, Bis(2,6-di-tert-butyl-4-methylphenyl)pentaerythritol diphosphite, 2,2'-Methylenebis(4,6-di-tert-butylphenyl) octyl phosphite, Tetrakis(2,4-di-tert-butylphenyl)[1,1'-biphenyl]-4,4'-diylbisphosphonite, 2,2'-Ethylidenebis(4,6-di-tert-butylphenyl) fluorophosphite, Dioctadecyl pentaerythritol diphosphonite, 2-[[2,4,8,10-Tetrakis(1,1-dimethylethyl)dibenzo[d,f]-[1,3,2]dioxaphosphepin-6-yl]oxy]-N,N-bis[2-[[2,4,8,10-tetrakis(1,1-dimethylethyl)dibenzo-[d,f][1,3,2]dioxaphosphepin-6-yl]oxy]ethyl]ethanamine (CAS-Nr. 80410-33-9), Bis(2,4-di-tert-butyl-6-methylphenyl) ethyl phosphite, 2,4,6-Tri-tert-butylphenyl- 2-butyl-2-ethyl-1,3-propanediol phosphite, Bis(2,4-dicumylphenyl) pentaerythritol diphosphite,
Hydroxylamine wie,
Amines, bis(hydrogenated tallow alkyl), oxidized ,
Sekundare Arylamine wie,
N-(2-Naphthyl)-N-phenylamine, 2,2,4-Trimethyl-1,2-dihydroquinoline polymer (CAS-Nr: 26780-96-1), N-2-Propyl-N'-phenyl-p-phenylenediamine, N-(1-Naphthyl)-N-phenylamine, (Benzenamine, N-phenyl-, reaction products with 2,4,4-trimethylpentene) (CAS-Nr. 68411-46-1), 4-(1-Methyl-1-phenylethyl)-N-[4-(1-methyl-1-phenylethyl)phenyl]aniline.

Lactone und Benzofuranone wie,
Irganox HP 136 (CAS Nr. 181314-48-7)
Thioether und Thioester wie,
Distearyl-3,3-thiodipropionate, Dilauryl 3,3'-thiodipropionate, Ditetradecylthiodipropionate, Di-n-octadecyl disulfide.

UV-Absorber wie,
(Methanone, [methylenebis(hydroxymethoxyphenylene)]bis[phenyl-), (Methanone, [1,6-hexanediylbis[oxy(2-hydroxy-4,1-phenylene)]]bis[phenyl-), 2-Benzoyl-5-methoxyphenol, 2,4-Dihydroxybenzophenone, 2,2'-Dihydroxy-4-methoxybenzophenone, 2-Hydroxy-4-octyloxybenzophenone, 2-Hydroxy-4-dodecyloxybenzophenone, 2-(2-Hydroxy-4-hexyloxyphenyl)-4,6-diphenyl-1,3,5-triazine, 2,4-Bis(2,4-dimethylphenyl)-6-(2-hydroxy-4-octyloxyphenyl)-1,3,5-triazine, 2-Ethoxy-2'-ethyloxalic acid bisanilide, N-(5-tert-Butyl-2-ethoxyphenyl)-N'-(2-ethylphenyl)-oxamide, Dimethyl (p-methoxybenzylidene)malonate, 2,2'-(1,4-Phenylene)bis[3,1-benzoxazin-4-one], N'-(4-Ethoxycarbonylphenyl)-N-methyl-N-phenylformamidine, 4-Methoxycinnamic acid 2-ethylhexyl ester, 4-Methoxycinnamic acid isoamyl-ester, 2-Phenylbenzimidazole-5-sulfonsäure, 2-Cyano-3,3-diphenylacrylic acid 2-ethylhexyl ester, 2-Ethylhexyl salicylate, 3-(4-Methylbenzylidene)bornan-2-one,
Chelatoren wie,
Ethylendiamintetraacetat (EDTA), Ethylendiamin,Acetylaceton, Nitrilotriessigsäure, Ethylenglycol-bis(ß-aminoethyl ether)-N,N-tetraessigsäure, 2,2'-Bipyridine, 4,4'-Dimethyl-2,2'-bipyridin, 2,2',6',2"-Terpyridin, ,4,4'Diphenyl-2,2'-bipyridin, 2,2'-Bipyridin-3,3'-diol, 1,10-Phenanthroline, 4-Methyl-1,10-phenanthroline, 5-Methyl-1,10-phenanthroline, 4,7-Dimethyl-1,10-phenanthroline, 5,6-Dimethyl-1,10-phenanthroline, 3,4,7,8-Tetramethyl-1,10-phenanthroline, 4,7-Diphenyl-1,10-phenanthroline, 2,4,7,9-Tetramethyl-1,10-phenanthroline, N,N,N',N'-Tetramethylethylendiamin, 2-Hydroxychinolin, 8-Hydroxychinolin, 2-Hydroxy-4-methyl-chinaldin, 5-Chlor-8-hydroxychinolin, 5,7-Dichlor-8-hydroxychinolin, 2,4-Chinolindiol, 2-Chinolinthiol, 8-Chinolinthiol, 8-Aminochinolin, 2,2'-Bichinolin, 2-Chinoxalinol, 3-Methyl-2-chinoxalinol, 2,3-Dihydroxychinoxaline, 2-Mercaptopyridin, 2-Dimethylaminopyridin, 1,2-Bis(Dimethylphosphino)ethan, 1,2-Bis-(diphenyl-phosphino)ethan, 1,3-bis(diphenylphosphino)propane, 1,4-Bis(diphenylphosphino)butan, Polyasparaginsäure, Iminodisuccinat.

Die erfindungsgemäßen Bindemittelformulierungen eignen sich insbesondere zur Anwendung als Anstrichmittel, insbesondere zur Verwendung als Farbe, Lack, Grundierung, Imprägnierung oder Lasur. Vorgenannte Verwendungen sind ebenfalls Gegenstand der Erfindung.

Die erfindungsgemäßen Bindemittelformulierungen enthaltend Übergangsmetalltrockner selbst zeigen gegenüber Bindemittelformulierungen, in denen die Iod enthaltenden Verbindungen, wie, insbesondere IPBC, nicht stabilisiert sind, keine Verlängerung der Trockenzeit wie sie häufig bei Zusatz von Stabilisatoren beobachtet wird.

Die Erfindung betrifft weiterhin die Verwendung der erfindungsgemäßen bioziden Mittel zum Schutz von technischen Materialien gegen Zerstörung oder Befall durch Mikroorganismen.

Die erfindungsgemäßen bioziden Mittel eignen sich zur bioziden Ausrüstung von technischen Materialien. Unter technischen Materialien sind im vorliegenden Zusammenhang nicht-lebende Materialien zu verstehen, die für die Verwendung in der Technik zubereitet worden sind. Beispielsweise handelt es sich bei den technischen Materialien um Klebstoffe, Leime, Papier und Karton, Textilien, Leder, Holz, Holzwerkstoffe, Anstrichmittel und Kunststoffartikel, Kühlschmierstoffe und andere Materialien, die von Mikroorganismen befallen oder zersetzt werden können.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Bakterien.

Es seien beispielsweise Mikroorganismen der folgenden Gattung genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.
Von der Erfindung sind auch die technischen Materialien enthaltend wenigstens eine Iod enthaltende Verbindung mit biozider Wirkung sowie ein Stickstoff enthaltendes Polymer umfasst.

Die Erfindung wird nachstehend anhand von Beispielen erläutert.

### Beispiele:

### Beispiel 1

20 g Trimethylolpropan-tris[3-(2-methyl-1-aziridinyl)propionat] (Crosslinker CX-100 von DSM) wurden in 50 ml Wasser vorgelegt und unter Rührung mit dem Magnetrührer mit 30 g Butyldiglycol versetzt. Anschließend wurde 6 h bei 80°C gerührt. Man erhielt eine nach dem Abkühlen klare, leicht gelbstichige Lösung.

### Bestimmung des Molekulargewichtes:

25 g der oben hergestellten Probe wurden bei 50 °C im Ölpumpenvakuum (ca. 0,35 mbar) vom Wasser befreit. Man erhielt 12,85 g eines hochviskosen Öls. 1 g dieses Öls wurde 3 mal mit je 5 g THF verrührt und der Rückstand über Nacht im Exsikkator getrocknet und mittels GPC (Standard: Polystyrol /PSS Polymerkit)) untersucht. Man identifizierte ein Polymer mit einem mittleren Molekulargewicht von 12,238 g/mol. In der THF Waschflüssigkeit konnte mit GC-MS nur Butyldiglycol als einzige Komponente nachgewiesen werden.

Aziridinfunktionalitäten konnten nicht nachgewiesen werden.

### Beispiel 2

20 g Trimethylolpropan-tris[3-(2-methyl-1-aziridinyl)propionat] (Crosslinker CX-100 von DSM) wurden in 20 ml Wasser vorgelegt und unter Rührung mit dem Magnetrührer mit 30 g Butyldiglycol versetzt. Anschließend wurde 6 h bei 80°C gerührt. Man erhielt eine nach dem Abkühlen klare, leicht gelbstichige Lösung.

### Brechungsindex n_{D} = 1,4115 (23 °C)

Mit quantitativer LC-MS konnte in dieser Lösung ein Anteil an dem Startprodukt Crosslinker CX-100 < 50 ppm gefunden werden.

### Beispiel 3: Herstellung eines erfindungsgemäßen bioziden Mittels

40 g Trimethylolpropan-tris[3-(2-methyl-1-aziridinyl)propionat] (Crosslinker CX-100 von DSM) wurden in 100 ml Wasser vorgelegt und unter Rührung mit dem Magnetrührer mit 60 g Butyldiglycol versetzt. Anschließend wurde 6 h bei 80°C gerührt. Man erhielt eine nach dem Abkühlen klare, leicht gelbstichige Lösung. Diese Lösung wurde unter Rühren mit weiteren 320 g Butyldiglycol und 120 g IPBC (Iodpropargylbutylcarbamat) versetzt und 45 Min. mit einem Magnetrührer gerührt. Man erhielt 640 g einer leicht gelben Lösung mit einem IPBC Gehalt von 18,8 Gew.-%.

150 g der oben hergestellten Lösung wurden unter Rühren mit 0,3 g Ameisensäure versetzt und noch 5 Min nachgerührt. Das IPBC haltige, stabilisierte biozide Mittel war hellgelb und klar.

### Brechungsindex n_{D} = 1,4375 (23 °C)

### Beispiel 4: Bindemittelformulierungen

Die gemäß den Beispielen 1, 2 und 3 erhaltenen Verbindungen bzw. Mittel wurden in einem typischen, Alkydharz enthaltenden Anstrichsystem (Alkydlasur A / Tabelle 1) in Gegenwart eines Übergangsmetalltrockners (Co) und eines Metalloxidpigmentes (Eisenoxid) eingebracht. Im Fall der Beispiele 1 und 2, die kein IPBC enthalten, wird zusätzlich noch ein IPBC Konzentrat aus Tabelle 2 zugegeben. Als Vergleich wird IPBC direkt (unstabilisertes IPBC) bzw. ein Konzentrat aus Tabelle 3 (stabilisertes IPBC gemäß EP 2236033) eingearbeitet. Die Zusammensetzung der fertigen Lasuren ist Tabelle 4 zu entnehmen. In allen Beispielen betragt die IPBC Konzentration in den Lasuren 0,7 %.

| **Tabelle 1** (Rezeptur Alkydlasur A) | | |
|---|---|---|
| | Inhaltsstoffe | Gehalt [Gew.-%] |
| Alkydlasur A | Vialkyd VAF 4349, 80 SD 60, Fa. Cytec | 22,5 |
| | Polares LösungsmittelTexanol, Fa. Eastman | 5,0 |
| | RheologieadditivBYK E411, Fa. BYK | 0,4 |
| | Shellsol D60, Fa. Shell Chemicals | 67,8 |
| | MK-Solcolor Eisenoxidrot 130M (Pigmentpräparation), Fa. MK Chemicals | 4,0 |
| | Octa-Soligen® 69 (enthält 6% Co), Fa. Borchers | 0,3 |

**Tabelle 2 - IPBC-Konzentrat (unstabilisiert)**

| | |
|---|---|
| IPBC | 21 Gew.-% |
| Texanol (2,2,4-Trimethyl-1,3-pentandiolmonoisobutyrat) | 79 Gew.-% |

**Tabelle 3 - IPBC/Aziridin-Konzentrat / Vergleich**

| | |
|---|---|
| IPBC | 30 Gew.-% |
| Crosslinker CX-100** | 15 Gew.-% |
| Rhodiasolv DIB* | 55 Gew.-% |

| | |
|---|---|
| *Gemisch bestehend aus Diisobutyladipat, Diisobutylglutarat, Diisobutylsuccinat, Fa. Rhodia. ** Trimethylolpropan-tris[3-(2-methyl-1-aziridinyl)propionat] | |

Zur Bestimmung der Stabilisierung wird ein beschleunigter Alterungstest durchgeführt. Hierzu wird das ausgerüstete Farbsystem in dicht schließende 200ml-Glasflaschen gefüllt, wobei nur eine minimale Restmenge Luft in dem Gebinde verbleibt, und bei 40°C gelagert. Die Ergebnisse sind der Tabelle 5 zu entnehmen.

| **Tabelle 4** - Rezeptur einer pigmentierten mit IPBC ausgerüsteten Alkydharz enthaltenden Lasur. | | | | | |
|---|---|---|---|---|---|
| Inhaltsstoffe | Alkydl. A-I [%] | Alkydl. A-II [%] | Alkydl. A-III [%] | Alkydl. A-IV [%] Vergleich 1 | Alkydl. A-V [%] Vergleich 2 |
| Alkydlasur aus Tabelle 1 | 95,4 | 95,84 | 96,2 | 99,3 | 97,67 |
| IPBC-Lösung in Texanol (21 % IPBC / 79 % Texanol; Tabelle 1) | 3,42¹⁾ | 3,27¹⁾ | - | - | - |
| Stabilisator aus Bsp. 1 | 1,18 | - | - | - | - |
| Stabilisator aus Bsp. 2 | | 0,89 | - | | |
| Konzentrat aus Bsp. 3 | | | 3,8¹⁾ | | |
| IPBC | - | - | - | 0,7¹⁾ | - |
| IPBC/Aziridin-Konzentrat (Tabelle 3) | - | - | - | - | 2,33¹⁾ |

| | | | | | |
|---|---|---|---|---|---|
| 1) entspricht jeweils 0,7 Gew.-% IPBC, bezogen auf die Lasur. | | | | | |

| **Tabelle 5** - Stabilität des IPBC in den Alkydlasuren A (-I) bis (-V) bei 40°C | | | | |
|---|---|---|---|---|
| | Restgehalt IPBC [%] bezogen auf den Startwert | | | |
| Alkydlasur | Start | 2 Wochen | 4 Wochen | 8 Wochen |
| A-I | 100 | - | 97 | 94 |
| A-II | 100 | - | 96 | 92 |
| A-III | 100 | - | 98 | 90 |
| A-IV1) | 100 | 96 | 52 | 0 |
| A-V2) | 100 | 100 | 80 | 0 |

| | | | | |
|---|---|---|---|---|
| 1) nicht stabilisierte Probe 2) Mit Aziridin stabilisiertes IPBC ohne Hydrolyse (gemäß EP 2 236 033 A) | | | | |

Aus Tabelle 5 wird deutlich, dass die Stickstoff enthaltenden Polymere im Hinblick auf die Stabilisierung von IPBC eine deutlich höhere Stabilität gegenüber der unstabilisierten Probe A-IV aufweisen. Auch gegenüber der mit unreagiertem Aziridin stabilisierten IPBC-Probe (Lasur A-V) zeigt sich noch eine deutliche Verbesserung.

## Patentansprüche

1. Verwendung von Stickstoff enthaltenden Polymeren zur Stabilisierung von Iod enthaltenden Verbindungen, **dadurch gekennzeichnet, dass** die Stickstoff enthaltenden Polymere solche sind, die durch Umsetzung von Aziridinen in Gegenwart von Wasser erhältlich sind.

2. Verfahren zur Stabilisierung von Iod enthaltenden Verbindungen durch Inkontaktbringen der Iod enthaltenden Verbindungen mit Stickstoff enthaltenden Polymeren, **dadurch gekennzeichnet, dass** die Stickstoff enthaltenden Polymere solche sind, die durch Umsetzung von Aziridinen in Gegenwart von Wasser erhältlich sind..

3. Verwendung nach Anspruch 1 und Verfahren nach Anspruch 2, **dadurch gekennzeichnet dass** die Iod enthaltenden Verbindungen Iodalkinylverbindungen sowie Verbindungen sind, in denen ein oder mehrere Iodatome an sp²-hybridisierte Kohlenstoffatome von olefinischen Doppelbindungen, oder an sp³-hybridisierte Kohlenstoffatome gebunden sind.

4. Verwendung und Verfahren nach Anspruch 3, **dadurch gekennzeichnet dass** Iod enthaltende Verbindungen biozide Wirksamkeit aufweisen und folgende sind: N-(C₁-C₁₂)-Alkyl-iodtetrazole, N-(C₆-C₁₅)-Aryl-iodtetrazole, N-(C₆-C₁₅)-Arylalkyl-iodtetrazole Diiodmethyl-p-tolylsulfon, Diiodmethyl-p-chlorphenylsulfon, 3-Brom-2,3-diiod-2-propenylalkohol, 2,3,3-Triiodallylalkohol, 4-Chlor-2-(2-chlor-2-methylpropyl)-5-[(6-iod-3-pyridinyl)methoxy]-3(2H)-pyridazinone (CAS-RN: 120955-77-3), Iodfenfos, 3-Iod-2-propinyl-2,4,5-trichlorphenylether, 3-Iod-2-propinyl-4-chlorphenylformal (IPCF), N-Iodpropargyloxycarbonyl-alanin, N-Iodpropargyloxycarbonyl-alanin-ethylester, 3-(3-Iodpropargyl)-benzoxazol-2-on, 3-(3-Iodpropargyl)-6-chlorbenzoxazol-2-on, 3-Iod-2-propinyl-alkohol, 4-Chlorphenyl-3-iodpropargylformal,3-Iod-2-propinyl-propyl-carbamat, 3-Iod-2-propinyl-butyl-carbamat (IPBC), 3-Iod-2-propinyl-m-chlorphenyl-carbamat, 3-Iod-2-propinyl-phenyl-carbamat, Di-(3-Iod-2-propinyl)hexyl-dicarbamat, 3-Iod-2-propinyloxyethanol-ethylcarbamat, 3-Iod-2-propinyl-oxyethanol-phenyl-carbamat, 3-Iod-2-propinyl-thioxo-thioethylcarbamat, 3-Iod-2-propinyl-carbaminsäureester (IPC), 3-Brom-2,3-diiod-2-propenylethylcarbamat, 3-Iod-2-propinyl-n-hexylcarbamat und 3-Iod-2-propinyl-cyclohexylcarbamat.

5. Verwendung nach einem der Ansprüche 1 oder 3 bis 4 und Verfahren nach einem der Ansprüche 2 bis 4 **dadurch gekennzeichnet dass** die Stickstoff enthaltenden Polymere besitzen ein gewichtsmittleres Molekulargewicht von mehr als 1,000 g/mol, vorzugsweise 2,000 bis 100,000 g/mol und besonders bevorzugt 3,000 und 60,000 g/mol bestimmt durch Gel-Permeations-Chromatographie gegen Polystyrol-Standard aufweisen.

6. Verwendung nach einem der Ansprüche 1 oder 3 bis 5 und Verfahren nach einem der Ansprüche 2 bis 5, **dadurch gekennzeichnet dass** die Stickstoff enthaltenden Polymere einen Stickstoffgehalt von 1 bis 20 Gew.-%, bevorzugt 2 bis 15 Gew.-% N, und besonders bevorzugt 5 bis 12 % Gew.-% N ermittelt durch Elementaranalyse aufwiesen.

7. Verwendung nach einem der Ansprüche 1 oder 3 bis 6 und Verfahren nach einem der Ansprüche 2 bis 6, **dadurch gekennzeichnet, dass** die Stickstoff enthaltenden Polymere solche sind, die zumindest eine, vorzugsweise mehrere beta-Aminoamin-Funktionen besitzt,

8. Verwendung nach einem der Ansprüche 1 oder 3 bis 7 und Verfahren nach einem der Ansprüche 2 bis 7, **dadurch gekennzeichnet, dass** die Aziridine Aziridinverbindungen der Formel (I) sind 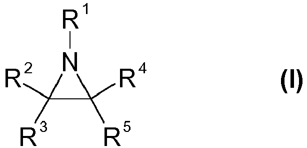 wobei
R¹ Wasserstoff, Alkyl oder Cycloalkyl, die jeweils unsubstituiert oder substituiert und/oder einfach- oder mehrfach ethylenisch ungesättigt sind, jeweils substituiertes oder unsubstituiertes Fullerenyl, Aryl, Alkoxy, Alkoxycarbonyl, Arylcarbonyl oder Alkanoyl bedeutet,
R², R³, R⁴ und R⁵ unabhängig voneinander die gleiche Bedeutung wie R¹ haben und zusätzlich unabhängig Halogen, Hydroxyl, Carboxyl, Alkylsulfonyl, Arylsulfonyl, Nitril, Isonitril bedeuten und
R² und R⁴ oder R³ und R⁵ gemeinsam mit den Kohlenstoffatomen, an die sie gebunden sind, einen 5- bis 10 gliedrigen carbocyclischen Ring bilden, der unsubstituiert oder substituiert und/oder einfach- oder mehrfach ethylenisch ungesättigt ist.

9. Biozide Mittel enthaltend zumindest
a) mindestens eine Iod enthaltende Verbindung mit biozider Wirkung
b) mindestens ein Stickstoff enthaltendes Polymer,
**dadurch gekennzeichnet, dass** die Stickstoff enthaltenden Polymere solche sind, wie sie in einem der Ansprüche 5 bis 8 und deren Rückbezügen aufeinander spezifiziert werden

10. Biozides Mittel nach Anspruch 9, **dadurch gekennzeichnet, dass** es weiterhin Säure enthält.

11. Biozides Mittel nach einem der Ansprüche 9 oder 10, **dadurch gekennzeichnet, dass** es weitere Wirkstoffe ausgewählt aus der Gruppe der antimikrobiell wirksamen Verbindungen, Fungizide, Bakterizide, Herbizide und Insektizide enthält.

12. Bindemittelformulierung, enthaltend
a) zumindest ein Bindemittel,
b) zumindest eine Iod enthaltende Verbindung mit biozider Wirkung und
c) zumindest ein Stickstoff enthaltendes Polymer,
**dadurch gekennzeichnet, dass** die Stickstoff enthaltenden Polymere solche sind, wie sie in einem der Ansprüche 5 bis 8 und deren Rückbezügen aufeinander spezifiziert werden.

13. Bindemittelformulierung nach Anspruch 12, **dadurch gekennzeichnet, dass** sie weiterhin zumindest einen Übergangsmetalltrockner enthält.

14. Verwendung der bioziden Mittel nach einem der Ansprüche 9 bis 12 zum Schutz von technischen Materialien gegen Zerstörung oder Befall durch Mikroorganismen.

## Claims

1. Use of nitrogen containing polymers for stabilizing iodine containing compounds, **characterized in that** the nitrogen containing polymers are polymers obtainable by reaction of aziridines in the presence of water.

2. Method for stabilizing iodine containing compounds by contacting the iodine containing compounds with nitrogen containing polymers, **characterized in that** the nitrogen containing polymers are polymers obtainable by reaction of aziridines in the presence of water.

3. Use according to Claim 1 and method according to Claim 2, **characterized in that** the iodine containing compounds are iodoalkynyl compounds and also compounds in which one or more iodine atoms are bonded to sp²-hybridized carbon atoms of olefinic double bonds or to sp³-hybridized carbon atoms.

4. Use and method according to Claim 3, **characterized in that** iodine containing compounds have biocidal activity and are as follows: N-(C₁-C₁₂)-alkyl-iodotetrazoles, N-(C₆-C₁₅)-aryl-iodotetrazoles, N-(C₆-C₁₅)-arylalkyl-iodotetrazoles, diiodomethyl p-tolyl sulphone, diiodomethyl p-chlorophenyl sulphone, 3-bromo-2,3-diiodo-2-propenyl alcohol, 2,3,3-triiodoallyl alcohol, 4-chloro-2-(2-chloro-2-methylpropyl)-5-[(6-iodo-3-pyridinyl)methoxy]-3(2H)-pyridazinone (CAS RN: 120955-77-3), iodofenphos, 3-iodo-2-propynyl 2,4,5-trichlorophenyl ether, 3-iodo-2-propynyl 4-chlorophenyl formal (IPCF), N-iodopropargyloxycarbonyl-alanine, N-iodopropargyloxycarbonyl-alanine ethyl ester, 3-(3-iodopropargyl)benzoxazol-2-one, 3-(3-iodopropargyl)-6-chlorobenzoxazol-2-one, 3-iodo-2-propynyl alcohol, 4-chlorophenyl 3-iodopropargyl formal, 3-iodo-2-propynyl propylcarbamate, 3-iodo-2-propynyl butylcarbamate (IPBC), 3-iodo-2-propynyl m-chlorophenylcarbamate, 3-iodo-2-propynyl phenylcarbamate, di-(3-iodo-2-propynyl)hexyl dicarbamate, 3-iodo-2-propynyloxyethanol ethylcarbamate, 3-iodo-2-propynyloxyethanol phenylcarbamate, 3-iodo-2-propynyl thioxothioethylcarbamate, 3-iodo-2-propynyl-carbamic ester (IPC), 3-bromo-2,3-diiodo-2-propenyl ethylcarbamate, 3-iodo-2-propynyl n-hexylcarbamate and 3-iodo-2-propynyl cyclohexylcarbamate.

5. Use according to any of Claims 1 and 3 to 4 and method according to any of Claims 2 to 4, **characterized in that** the nitrogen containing polymers possess a weight-average molecular weight of more than 1000 g/mol, preferably 2000 to 100,000 g/mol and more preferably 3000 to 60,000 g/mol determined by gel permeation chromatography against polystyrene standard.

6. Use according to any of Claims 1 and 3 to 5 and method according to any of Claims 2 to 5, **characterized in that** the nitrogen containing polymers have a nitrogen content of 1% to 20% by weight, preferably 2% to 15% by weight N, and more preferably 5% to 12% by weight N, determined by elemental analysis.

7. Use according to any of Claims 1 and 3 to 6 and method according to any of Claims 2 to 6, **characterized in that** the nitrogen containing polymers are polymers possessing at least one, preferably two or more, beta-aminoamine functions.

8. Use according to any of Claims 1 and 3 to 7 and method according to any of Claims 2 to 7, **characterized in that** the aziridines are aziridine compounds of the formula (I) 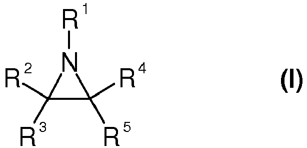 where
R¹ is hydrogen, alkyl or cycloalkyl, each of which is unsubstituted or substituted and/or mono- or polyethylenically unsaturated, or in each case substituted or unsubstituted fullerenyl, aryl, alkoxy, alkoxycarbonyl, arylcarbonyl or alkanoyl,
R², R³, R⁴ and R⁵ independently of one another have the same definition as R¹ and additionally independently are halogen, hydroxyl, carboxyl, alkylsulphonyl, arylsulphonyl, nitrile, isonitrile, and
R² and R⁴ or R³ and R⁵, together with the carbon atoms to which they are attached, form a 5- to 10-membered carbocyclic ring which is unsubstituted or substituted and/or mono- or polyethylenically unsaturated.

9. Biocidal compositions comprising at least
a) at least one iodine containing compound having a biocidal action
b) at least one nitrogen containing polymer,
**characterized in that** the nitrogen containing polymers are polymers as specified in any of Claims 5 to 8 and their dependency references to one another.

10. Biocidal composition according to Claim 9, **characterized in that** it further comprises acid.

11. Biocidal composition according to either of Claims 9 and 10, **characterized in that** it comprises further active ingredients selected from the group of antimicrobially active compounds, fungicides, bactericides, herbicides and insecticides.

12. Binder formulation comprising
a) at least one binder,
b) at least one iodine containing compound with biocidal action and
c) at least one nitrogen containing polymer,
**characterized in that** the nitrogen containing polymers are polymers as specified in any of Claims 5 to 8 and their dependency references to one another.

13. Binder formulation according to Claim 12, **characterized in that** it further comprises at least one transition metal dryer.

14. Use of the biocidal compositions according to any of Claims 9 to 12 for protecting industrial materials against destruction or infestation by microorganisms.

## Revendications

1. Utilisation de polymères contenant de l'azote pour la stabilisation de composés contenant de l'iode, **caractérisée en ce que** les polymères contenant de l'azote sont des polymères qui peuvent être obtenus par mise en réaction d'aziridines en présence d'eau.

2. Procédé de stabilisation de composés contenant de l'iode par mise en contact des composés contenant de l'iode avec des polymères contenant de l'azote, **caractérisé en ce que** les polymères contenant de l'azote sont des polymères qui peuvent être obtenus par mise en réaction d'aziridines en présence d'eau.

3. Utilisation selon la revendication 1 et procédé selon la revendication 2, **caractérisés en ce que** les composés contenant de l'iode sont des composés d'iodoalcynyle et des composés dans lesquels un ou plusieurs atomes d'iode sont reliés à des atomes de carbone à hybridation sp² de doubles liaisons oléfiniques ou à des atomes de carbone à hybridation sp³.

4. Utilisation et procédé selon la revendication 3, **caractérisés en ce que** les composés contenant de l'iode présentent une activité biocide et sont les suivants : N-alkyle en (C₁-C₁₂)-iodotétrazole, N-aryle en (C₆-C₁₅)-iodotétrazole, N-arylalkyle en (C₆-C₁₅)-iodotétrazole, diiodométhyl-p-tolylsulfone, diiodométhyl-p-chlorophénylsulfone, alcool 3-bromo-2,3-diiodo-2-propénylique, alcool 2,3,3-triiodoallylique, 4-chloro-2-(2-chloro-2-méthylpropyl)-5-[(6-iodo-3-pyridinyl)méthoxy]-3(2H)-pyridazinone (n° CAS : 120955-77-3), iodfenfos, éther 3-iodo-2-propynyl-2,4,5-trichlorophénylique, 3-iodo-2-propynyl-4-chlorophénylformal (IPCF), N-iodopropargyloxycarbonyl-alanine, ester N-iodopropargyloxycarbonyl-alanine-éthylique, 3-(3-iodopropargyl)-benzoxazol-2-one, 3-(3-iodopropargyl)-6-chlorobenzoxazol-2-one, alcool 3-iodo-2-propynylique, 4-chlorophényl-3-iodopropargylformal, carbamate de 3-iodo-2-propynyl-propyle, carbamate de 3-iodo-2-propynyl-butyle (IPBC), carbamate de 3-iodo-2-propynyl-m-chlorophényle, carbamate de 3-iodo-2-propynyl-phényl, dicarbamate de di-(3-iodo-2-propynyl)hexyle, carbamate de 3-iodo-2-propynyloxyéthanol-éthyle, carbamate de 3-iodo-2-propynyl-oxyéthanol-phényle, carbamate de 3-iodo-2-propynyl-thioxo-thioéthyle, ester de l'acide 3-iodo-2-propynyl-carbamique (IPC), carbamate de 3-bromo-2,3-diiodo-2-propényléthyle, carbamate de 3-iodo-2-propynyl-n-hexyle et carbamate de 3-iodo-2-propynyl-cyclohexyle.

5. Utilisation selon l'une quelconque des revendications 1 ou 3 à 4 et procédé selon l'une quelconque des revendications 2 à 4, **caractérisés en ce que** les polymères contenant de l'azote présentent un poids moléculaire moyen en poids de plus de 1 000 g/mol, de préférence de 2 000 à 100 000 g/mol et de manière particulièrement préférée de 3 000 à 60 000 g/mol, déterminé par chromatographie par perméation de gel contre un étalon polystyrène.

6. Utilisation selon l'une quelconque des revendications 1 ou 3 à 5 et procédé selon l'une quelconque des revendications 2 à 5, **caractérisés en ce que** les polymères contenant de l'azote présentent une teneur en azote de 1 à 20 % en poids, de préférence de 2 à 15 % en poids de N, et de manière particulièrement préférée de 5 à 12 % en poids de N, déterminée par analyse élémentaire.

7. Utilisation selon l'une quelconque des revendications 1 ou 3 à 6 et procédé selon l'une quelconque des revendications 2 à 6, **caractérisés en ce que** les polymères contenant de l'azote sont des polymères qui présentent au moins une, de préférence plusieurs fonctions bêta-aminoamine.

8. Utilisation selon l'une quelconque des revendications 1 ou 3 à 7 et procédé selon l'une quelconque des revendications 2 à 7, **caractérisés en ce que** les aziridines sont des composés d'aziridine de formule (I) 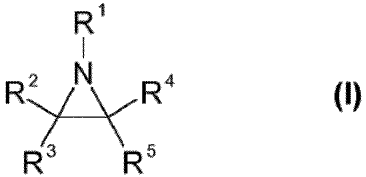 dans laquelle
R¹ signifie hydrogène, alkyle ou cycloalkyle, qui sont chacun non substitués ou substitués et/ou mono- ou polyéthyléniquement insaturés ; fullerényle, aryle, alcoxy, alcoxycarbonyle, arylcarbonyle ou alcanoyle, chacun substitué ou non substitué,
R², R³, R⁴ et R⁵ ont indépendamment les uns des autres la même signification que R¹ et signifient en outre indépendamment halogène, hydroxyle, carboxyle, alkylsulfonyle, arylsulfonyle, nitrile, isonitrile, et
R² et R⁴ ou R³ et R⁵ forment ensemble avec les atomes de carbone auxquels ils sont reliés un cycle carbocyclique de 5 à 10 éléments, qui est non substitué ou substitué et/ou mono- ou polyéthyléniquement insaturé.

9. Agents biocides, contenant au moins :
a) au moins un composé contenant de l'iode ayant une activité biocide
b) au moins un polymère contenant de l'azote,
**caractérisés en ce que** les polymères contenant de l'azote sont des polymères tels que spécifiés dans l'une quelconque des revendications 5 à 8 et leurs références entre elles.

10. Agent biocide selon la revendication 9, **caractérisé en ce qu'**il contient en outre un acide.

11. Agent biocide selon l'une quelconque des revendications 9 ou 10, **caractérisé en ce qu'**il contient d'autres agents actifs choisis dans le groupe constitué par les composés à action antimicrobienne, les fongicides, les bactéricides, les herbicides et les insecticides.

12. Formulation de liant, contenant :
a) au moins un liant,
b) au moins un composé contenant de l'iode à activité biocide et
c) au moins un polymère contenant de l'azote,
**caractérisée en ce que** les polymères contenant de l'azote sont des polymères tels que spécifiés dans l'une quelconque des revendications 5 à 8 et leurs références entre elles.

13. Formulation de liant selon la revendication 12, **caractérisée en ce qu'**elle contient en outre au moins un agent siccatif à base de métal de transition.

14. Utilisation des agents biocides selon l'une quelconque des revendications 9 à 12 pour la protection de matériaux techniques contre la destruction ou l'infestation par des microorganismes.
